(19) Europäisches Patentamt
European Patent Office
Office européen des brevets

(11) EP 1 149 074 B1

(12) EUROPEAN PATENT SPECIFICATION

(45) Date of publication and mention of the grant of the patent: **20.10.2004 Bulletin 2004/43**

(21) Application number: **00905787.8**

(22) Date of filing: **27.01.2000**

(51) Int Cl.[7]: **C07C 323/49**, C07C 327/06, C07D 295/12, C07F 9/53, A61K 31/18, A61P 19/02, A61P 37/00, C07C 309/42, C07C 309/87

(86) International application number: **PCT/US2000/002143**

(87) International publication number: **WO 2000/044716 (03.08.2000 Gazette 2000/31)**

(54) **ACETYLENIC SULFONAMIDE THIOL TACE INHIBITORS**

ACETYLENISCHE SULFONAMID-THIOL-TACE-INHIBITOREN

THIOLS SULFONAMIDE ACETYLENIQUES UTILES COMME INHIBITEURS DE L'ENZYME DE CONVERSION DU TNF-ALPHA(TACE)

(84) Designated Contracting States:
**AT BE CH CY DE DK ES FI FR GB GR IE IT LI LU MC NL PT SE**
Designated Extension States:
**AL LT LV RO SI**

(30) Priority: **27.01.1999 US 238393**

(43) Date of publication of application: **31.10.2001 Bulletin 2001/44**

(73) Proprietor: **Wyeth Holdings Corporation**
**Madison, NJ 07940 (US)**

(72) Inventors:
- **LEVIN, Jeremy, Ian**
  **New City, NY 10956 (US)**
- **CHEN, James, Ming**
  **Bedminster, NJ 07921 (US)**

(74) Representative: **Wileman, David Francis, Dr. et al**
**c/o Wyeth Laboratories**
**Huntercombe Lane South**
**Taplow Maidenhead Berkshire SL6 OPH (GB)**

(56) References cited:
**WO-A-98/03164**
**WO-A-98/03166**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 149 074 B1

Printed by Jouve, 75001 PARIS (FR)

## Description

This invention relates to acetylenic aryl sulfonamide thiols which act as inhibitors of TNF-α converting enzyme (TACE). The compounds of the present invention are useful in disease conditions mediated by TNF-α, such as rheumatoid arthritis, osteoarthritis, sepsis, AIDS, ulcerative colitis, multiple sclerosis, Crohn's disease and degenerative cartilage loss.

## BACKGROUND OF THE INVENTION

TNF-α converting enzyme (TACE) catalyzes the formation of TNF-α from membrane bound TNF-α precursor protein. TNF-α is a pro-inflammatory cytokine that is believed to have a role in rheumatoid arthritis [Shire, M. G.; Muller, G. W. *Exp. Opin. Ther. Patents* **1998,** *8(5)*, 531; Grossman, J. M.; Brahn, E. *J. Women's Health* **1997,** *6(6),* 627; Isomaki, P.; Punnonen, J. *Ann. Med.* **1997,** *29*, 499; Camussi, G.; Lupia, E. *Drugs,* **1998,** *55(5)*, 613.] septic shock [Mathison, *et. al*. *J. Clin. Invest.* **1988,** *81,* 1925; Miethke, *et. al. J. Exp. Med*. **1992,** *175,* 91.], graft rejection [Piguet, P. F.; Grau, G. E.; *et. al. J. Exp. Med*. **1987,** *166,* 1280.], cachexia [Beutler, B.; Cerami, A. *Ann. Rev. Biochem*. **1988,** *57,* 505.], anorexia, inflarnmation [Ksontini, R,; MacKay, S. L. D.; Moldawer, L. L. *Arch. Surg.* **1998,** *133*, 558.], congestive heart failure [Packer, M. *Circulation,* **1995,** *92(6),* 1379; Ferrari, R.; Bachetti, T.; et. al. *Circulation,* **1995,** *92(6),* 1479.], post-ischaemic reperfusion injury, inflammatory disease of the central nervous system, inflammatory bowel disease, insulin resistance [Hotamisligil, G. S.; Shargill, N. S.; Spiegelman, B. M.; *et. al. Science,* **1993,** *259,* 87.] and HIV infection [Peterson, P. K.; Gekker, G.; *et. al. J. Clin. Invest.* **1992,** *89*, 574; Pallares-Trujillo, J.; Lopez-Soriano, F. J. Argiles, J. M. *Med. Res. Reviews*, **1995,** *15(6),* 533.]], in addition to its well-documented antitumor properties [Old, L. *Science.,* **1985,** *230,* 630.]. For example, research with anti-TNF-α antibodies and transgenic animals has demonstrated that blocking the formation of TNF-α inhibits the progression of arthritis [Rankin, E.C.; Choy, E.H.; Kassimos, D.; Kingsley, G.H.; Sopwith, A.M.; Isenberg, D.A.; Panayi, G.S. *Br. J. Rheumatol.* **1995,** *34,* 334; *Pharmaprojects,* **1996,** Therapeutic Updates *17 (Oct.),* aul97-M2Z.]. This observation has recently been extended to humans as well as described in "TNF-α in Human Diseases", *Current Pharmaceutical Design,* **1996,** *2,* 662.,

It is expected that small molecule inhibitors of TACE would have the potential for treating a variety of disease states. Although a variety of TACE inhibitors are known, many of these molecules are peptidic and peptide-like which suffer from bioavailability and pharmacokinetic problems. In addition, many of these molecules are non-selective, being potent inhibitors of matrix metalloproteinases and, in pariticular, MMP-1. Inhibition of MMP-1 (collagenase 1) has been postulated to cause joint pain in clinical trials of MMP inhibitors [*Scrip,* **1998,** *2349,* 20] Long acting, selective, orally bioavailable non-peptide inhibitors of TACE would thus be highly desirable for the treatment of the disease states discussed above.

U. S. patents 5,455,258, 5,506,242, 5,552,419, 5,770,624, and 5,817,822 as well as European patent application EP606,046A1 and WIPO international publications WO9600214 and WO9722587 disclose non-peptide inhibitors of matrix metalloproteinases and/or TACE of which the aryl sulfonamide hydroxamic acid shown below is representative. Additional publications disclosing sulfonamide based MMP inhibitors which are variants of the sulfonamide-hydroxamate shown below, or the analogous sulfonamide-carboxylates, are European patent applications EP-757037-Al and EP-757984-Al and WIPO international publications WO9535275, WO9535276, WO9627583, WO9719068, WO9727174, WO9745402, WO9807697, WO9831664, WO9833768, WO9839313, WO9839329, WO9842659 and WO9843963. The discovery of this type of MMP inhibitor is further detailed by MacPherson, *et. al.* in *J. Med. Chem.,* (1997), 40, 2525 and Tamura, *et. al.* in *J. Med. Chem.* (1998), 41, 640.

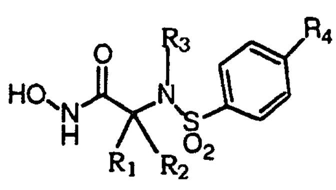

Publications disclosing β-sulfonamide-hydroxamate inhibitors of MMPs and/or TACE in which the carbon alpha to the hydroxamic acid has been joined in a ring to the sulfonamide nitrogen, as shown below, include U. S. patent 5,753,653, WIPO international publications WO9633172, WO9720824, WO9827069, WO9808815, WO9808822, WO9808823, WO9808825, WO9834918, WO9808827, Levin, *et. al. Bioorg. & Med Chem. Letters* **1998,** *8*, 2657 and Pikul, *et. al. J. Med Chem.* **1998,** *41,* 3568.

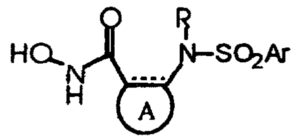

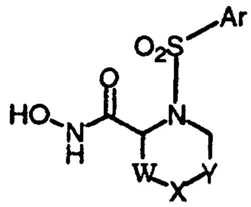

The patent applications DE19,542,189-A1, WO9718194, and EP803505 disclose additional examples of cyclic sulfonamides as MMP and/or TACE inhibitors. In this case the sulfonamide-containing ring is fused to a aromatic or heteroaromatic ring.

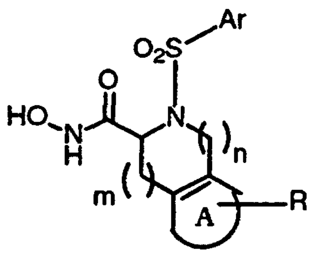

Examples of sulfonamide hydroxamic acid MMP/TACE inhibitors in which a 2 carbon chain separates the hydroxamic acid and the sulfonamide; nitrogen, as shown below, are disclosed in WIPO international publications WO9816503, WO9816506, WO9816514 and WO9816520 and U. S. patent 5,776,961.

Analogous to the sulfonamides are the phosphinic acid amide hydroxamic acid MMP/TACE inhibitors, exemplified by the structure below, which have been disclosed in WIPO international publication WO9808853.

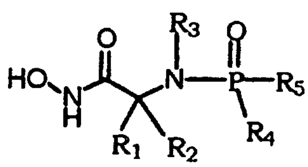

Sulfonamide MMP/TACE inhibitors in which a thiol is the zinc chelating group, as shown below, have been disclosed in WIPO international application 9803166.

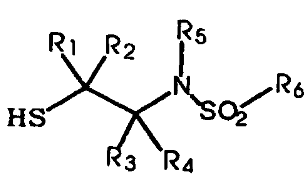

It is an object of this invention to disclose aryl sulfonamide hydroxamic acid MMP/TACE inhibitors in which the sulfonyl aryl group is para-substituted with a substituted butynyl moiety or a propargylic ether, amine or sulfide.

## SUMMARY OF THE INVENTION

The invention provides TACE and MMP inhibitors having the formula **B:**

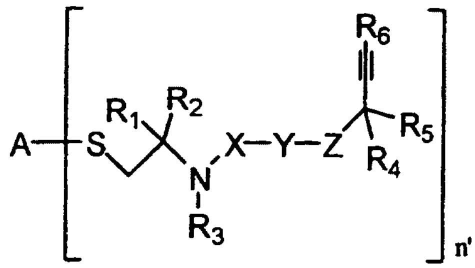

**B**

wherein n' = 1 or 2;
and when n' = 2, then A is absent (i.e. a disulfide);
and when n' = 1, then A is H or $R_{14}C(=W)$- ; in which formula:

W is oxygen or sulfur;

X is $SO_2$ or -P(O)-$R_{10}$;

Y is aryl or heteroaryl as defined below, with the proviso that X and Z may not be bonded to adjacent atoms of Y;

Z is O, NH, $CH_2$ or S;

$R_1$ is hydrogen, aryl, alkyl of 1-6 carbon atoms, alkenyl of 2-6 carbon atoms, alkynyl of 2-6 carbon atoms;

$R_2$ is hydrogen, aryl or heteroaryl as defined below, cycloalkyl of 3-6 carbon atoms, -$C_5$-$C_8$-cycloheteroalkyl, alkyl of 1-6 carbon atoms, alkenyl of 2-6 carbon atoms, alkynyl of 2-6 carbon atoms, or $CONR_8R_9$;

or $R_1$ and $R_2$, together with the atom to which they are attached, may form a ring wherein $R_1$ and $R_2$ represent a divalent moiety of the formula:

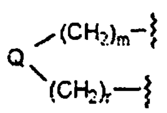 ;

wherein
Q = a carbon-carbon single or double bond, O, S, SO, -N-$R_{11}$, or - $CONR_{15}$;
m = 1-3;
r = 1 or 2, with the proviso that when Q is a bond, r is equal to 2;

Aryl is phenyl or naphthyl optionally substituted by one to two substituents selected from $R_7$, where $R_7$ is as defined below;

Heteroaryl is defined as

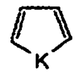, 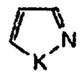, 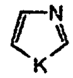, 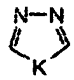, 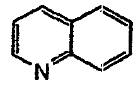,

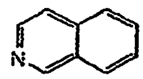, 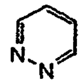; 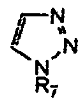, 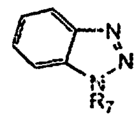,

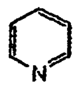, 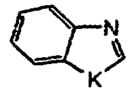, 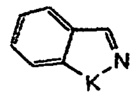, 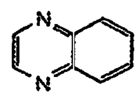,

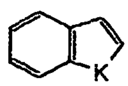, 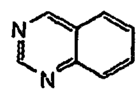, 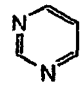, or 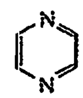;

optionally mono- or di- substituted by $R_7$, wherein K is defined as O, S or $-NR_{15}$;

$R_3$ is hydrogen or alkyl of 1-6 carbon atoms;

or $R_1$ and $R_3$, together with the atoms to which they are attached, may form a 5 to 8 membered ring wherein $R_1$ and $R_3$ represent divalent moieties of the formulae:

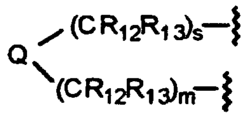 ; and 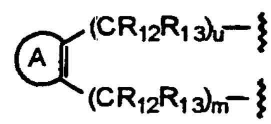

wherein Q and m are as defined above;
A is aryl or heteroaryl;
s is 0-3;
u is 1-4;

$R_4$ and $R_5$ are each, independently, hydrogen, alkyl of 1-6 carbon atoms, -CN, - CCH;

$R_6$ is hydrogen, aryl, heteroaryl, alkyl of 1-6 carbon atoms, alkenyl of 2-6 carbon atoms, alkynyl of 2-6 carbon atoms, cycloalkyl of 3-6 carbon atoms or-$C_5$-$C_8$-cycloheteroalkyl as defined below;

$R_7$ is hydrogen, halogen, alkyl of 1-6 carbon atoms; alkenyl of 2-6 carbon atoms; alkynyl of 2-6 carbon atoms, cycloalkyl of 3-6 carbon atoms, - $OR_8$, -CN, $-COR_8$, perfluoroalkyl of 1-4 carbon atoms, -O-perfluoroalkyl of 1-4 carbon atoms, $-CONR_8R_9$, $-S(O)_nR_8$, - $OPO(OR_8)OR_9$, $-PO(OR_8)R_9$, $-OC(O)NR_8R_9$, $-C(O)NR_8OR_9$, $-COOR_8$, - $SO_3H$, $-NR_8R_9$, $-N[(CH_2)_2]_2NR_8$, $-NR_8COR_9$, $-NR_8COOR_9$, $-SO_2NR_8R_9$, - $NO_2$, $-N(R_8)SO_2R_9$, $-NR_8CONR_8R_9$, $-NR_8C(=NR_9)NR_8R_9$, - $NR_8C(=NR_9)N(C=OR_8)R_9$, $-NR_8C(=NR_9)N(SO2R_8)R_9$, tetrazol-5-yl, $-SO_2NHCN$, $-SO_2NHCONR_8R_9$, phenyl, heteroaryl as defined above, or -$C_5$-$C_8$-cycloheteroalkyl as defined below;
wherein $-NR_8R_9$ may form a pyrrolidine, piperidine, morpholine, thiomorpholine, oxazolidine, thiazolidine, pyrazolidine, piperazine, or azetidine ring;

wherein $-C_5-C_8$-cycloheteroalkyl is defined as

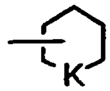 , 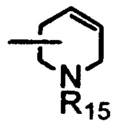 , 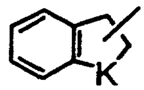 ,

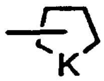 , 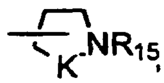 , 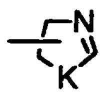 , 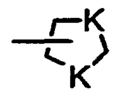 ,

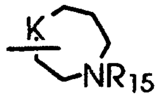 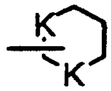 , 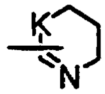 , or 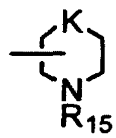 ;

wherein K is defined as above;

$R_8$ and $R_9$ are each, independently, hydrogen, alkyl of 1-6 carbon atoms, cycloalkyl of 3-6 carbon atoms, aryl, heteroaryl or $-C_5-C_8$-cyclohetero-alkyl;

$R_{10}$ is alkyl of 1-6 carbon atoms, cycloalkyl of 3-6 carbon atoms, aryl or heteroaryl as defined above;

$R_{11}$ is hydrogen, alkyl of 1-6 carbon atoms, cycloalkyl of 3-6 carbon atoms, aryl, heteroaryl, $-S(O)_nR_8$, $-COOR_8$, $-CONR_8R_9$, $-SO_2NR_8R_9$ or $-COR_8$;

$R_{12}$ and $R_{13}$ are independently selected from H, $-OR_8$, $-NR_8R_9$, alkyl of 1-6 carbon atoms, alkenyl of 2-6 carbon atoms, alkynyl of 2-6 carbon atoms, cycloalkyl of 3-6 carbon atoms, aryl, heteroaryl, $-COOR_8$; $-CONR_8R_9$; or $R_{12}$ and $R_{13}$ together form a $-C_3-C_6$-cycloalkyl of 3-6 carbon atoms or a $-C_5-C_8$-cycloheteroalkyl ring; or $R_{12}$ and $R_{13}$, together with the carbon to which they are attached, form a carbonyl group;
with the proviso that $R_{10}$ and $R_{12}$ or $R_{11}$ and $R_{12}$ may form a cycloheteroalkyl ring, wherein cycloheteroalkyl is as defined above, when they are attached to adjacent atoms;

$R_{14}$ is $-OR_8$, $-NR_8R_9$, alkyl of 1-6 carbon atoms, cycloalkyl of 3-6 carbon atoms, aryl or heteroaryl;

$R_{15}$ is hydrogen, aryl, heteroaryl, alkyl of 1-6 carbon atoms or cycloalkyl of 3-6 carbon atoms;

and n is 0-2;

or a pharmaceutically acceptable salt thereof.

This invention provides a process for preparing compounds of formula B as defined above which comprises one of the following:

a) reacting a compound of formula V:

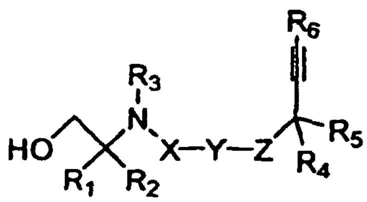

(V)

wherein $R_1$-$R_6$, X, Y and Z are defined above, with a compound of formula $R_{14}C(W)SH$ under conditions suitable for the Mitsunobu reaction to give a corresponding compound of formula B wherein n'=1 and A is $R_{14}C(=W)$-;
or
b) reacting a compound of formula VI:

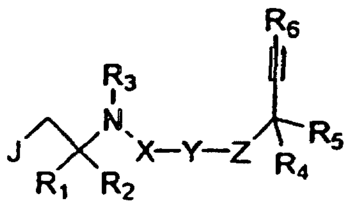

(VI)

wherein $R_1$-$R_6$, X, Y and Z are defined above, and J is a leaving group eg halogen such as chlorine, an organic sulphonyloxy group such as mesylate or tosylate or triflate , with a compound of formula A'SH where A' is an alkali metal or a $R_{14}C(W)$-group or a salt thereof to give a corresponding compound of formula **B** wherein n'=1 and A is H or $R_{14}C(=W)$-;
or
c) reacting a compound of formula VII:

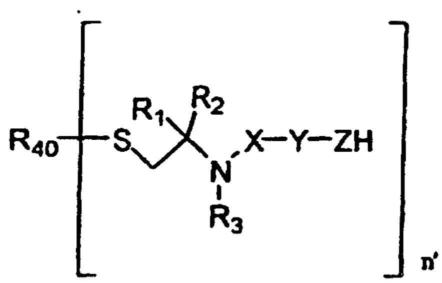

(VII)

wherein $R_1$, $R_2$, $R_3$, X, Y, Z and n' is as defined above, and when n'=1 $R_{40}$ is H or $R_{14}C(=W)$-, and when n'=2, $R_{40}$ is absent (i.e. a disulfide), with a compound of formula **VIII:**

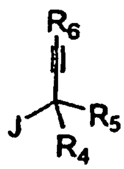

(VIII)

wherein $R_4$, $R_5$, and $R_6$ are as defined above and J is a leaving group such as described above, to give a corresponding compound of formula **B**;

or

d) hydrolysing a compound of formula **B** wherein $R_1$-$R_6$, X, Y and Z are defined above, n'=1 and A is $R_{14}C(=W)$- (i.e. formula **1b**), to give a corresponding compound of formula **B** wherein A is H;

or

e) reducing a compound of formula **B** wherein $R_1$-$R_6$, X, Y and Z are defined above, n'=1 and A is $R_{14}C(=W)$ or n' is 2 (i.e. formula **1c**), to give a corresponding compound of formula **B** wherein **A** is **H**;

or

f) oxidising a compound of formula **B** wherein $R_1$-$R_6$, X, Y and Z are defined above, n'=1 and A is H, to give a corresponding compound of formula **B** wherein n'=2;

or

g) resolving a mixture (e.g racemate) of optically active isomers of a compound of formula **B** to isolate one enantiomer or diastereomer substantially free of the other enantiomer or diastereomers;

or

h) acidifying a basic compound of formula **B** with a pharmaceutically acceptable acid to give a pharmaceutically acceptable salt.

With regard to process a), this can be carried out under standard conditions known for carrying out the Mitsunobu Reaction (see Merck Index 12th Edition ONR-61 and references therein) by using suitable reagents such as dialkyl azodicarboxylate, trialkyl- or triaryl-phosphines and for example thioacetic acid.

Process b) can be carried out by processes known in the art suitable for thiolation or thioesterification.

Process c) can be carried out by processes known in the art suitable for alkylation , where J is a suitable leaving group such as a halide, or an organic sulphonyloxy group, e.g. mesylate, tosylate or triflate.

Process d) can be carried out by processes known in the art, such as using sodium methoxide.

Process e) can be carried out by using the appropriate reducing agent, such as sodium borohydride

Process f), forming the corresponding disulfide, can be achieved by known processes in the art for oxidation such as the use of air or oxygen.

With regard to process g) standard separation techniques may be used to isolate particular enantiomeric or diastereomeric forms. For example a racemic mixture may be converted to a mixture of optically active diastereoisomers by reaction with a single enantiomer of a 'resolving agent' (for example by diastereomeric salt formation or formation of a covalent bond). The resulting mixture of optically active diastereoisomers may be separated by standard techniques (e.g crystallisation or chromatography) and individual optically active diastereoisomers then treated to remove the 'resolving agent' thereby releasing the single enantiomer of the compound of the invention. Chiral chromatography (using a chiral support, eluent or ion pairing agent) may also be used to separate enantiomeric mixtures directly.

The compounds of formula B may be isolated in the form of a salt of a pharmaceutically acceptable acid, e. g. an organic or inorganic acid by treatment with an acid such as described above."

The invention is further directed to a process for making compounds of structure **B** involving one or more reactions for preparing intermediates as follows:

1) converting a compound of formula **I,** or a salt or solvate thereof,

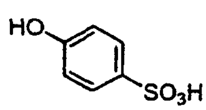

**I**

into a compound of formula **II**

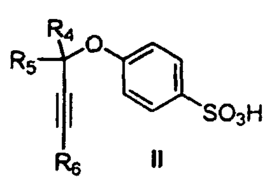

**II**

2) converting a compound of formula **II** above, or a salt or solvate thereof, to a compound of formula **III:**

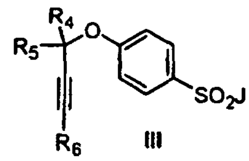

III

wherein J is fluorine, bromine, chlorine, 1,2,4-triazolyl, benzotriazolyl or imidazolyl, and $R_4$, $R_5$ and $R_6$ are as defined above.

The invention is still further directed to a process for making compounds of structure **B** involving one or more reactions for preparing intermediates as follows:

1) converting phenol, or a salt or solvate thereof, into a compound of formula **IV:**

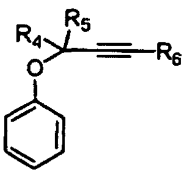

**IV**

2) converting a compound of formula **IV** above, or a salt or solvate thereof, to a compound of formula **II** above.

Alkyl, alkenyl, alkynyl, and perfluoroalkyl include both straight chain as well as branched moieties. The definitions of alkyl, alkenyl, alkynyl, cycloalkyl and phenyl include alkyl, alkenyl, alkynyl, cycloalkyl and phenyl moieties which are unsubstituted (carbons bonded to hydrogen, or other carbons in the chain or ring) or may be mono- or poly-substituted with $R_7$. When a moiety contains more than one substituent with the same designation (i.e., alkyl tri-substituted with R,) each of those substituents ($R_7$ in this case) may be the same or different. Halogen means bromine, chlorine, fluorine, and iodine.

The compounds of this invention may contain an asymmetric carbon atom and some of the compounds of this invention may contain one or more asymmetric centers and may thus give rise to optical isomers and diastereomers. While shown without respect to stereochemistry, the present invention includes such optical isomers and diastereomers; as well as the racemic and resolved. enantiomerically pure R and S stereoisomers; as well as other mixtures of the R and S stereoisomers and pharmaceutically acceptable salts thereof. It is recognized that one optical isomer, including diastereomer and enantiomer. or stereoisomer may have favorable properties over the other. Thus when disclosing and claiming the invention, when one racemic mixture is disclosed, it is clearly contemplated that both optical isomers, including diastereomers and enantiomers, or stereoisomers substantially free of the other are disclosed and claimed as well.

Pharmaceutically acceptable salts can be formed from organic and inorganic acids, for example, acetic, propionic, lactic, citric, tartaric, succinic, fumaric, maleic, malonic, mandelic, malic, phthalic, hydrochloric, hydrobromic, phosphoric, nitric, sulfuric, methanesulfonic, naphthalenesulfonic, benzenesulfonic, toluenesulfonic, camphorsulfonic, and similarly known acceptable acids when a compound of this invention contains a basic moiety. Salts may also be formed from organic and inorganic bases, preferably alkali metal salts, for example, sodium, lithium, or potassium, when a compound of this invention contains an acidic moiety.

Preferred compounds of the invention are those of formula **B** wherein n'=1, A is H, and Y is phenyl or a pharmaceutically acceptable salt thereof.

Preferred compounds of the invention are those of formula **B** wherein X is $SO_2$; or a pharmaceutically acceptable salt thereof.

Preferred compounds of the invention are those of formula B wherein Z is oxygen; or a pharmaceutically acceptable salt thereof.

Preferred compounds of the invention are those of formula **B** wherein $R_4$ and $R_5$ are hydrogen; or a pharmaceutically acceptable salt thereof.

Preferred compounds of the invention are those of formula **B** wherein $R_6$ is $-CH_2OH$ or methyl; or a pharmaceutically acceptable salt thereof.

Preferred compounds of the invention are those of formula **B** wherein $R_1$ is hydrogen, such that this compound has the absolute stereochemistry as shown in structure **1a** below.

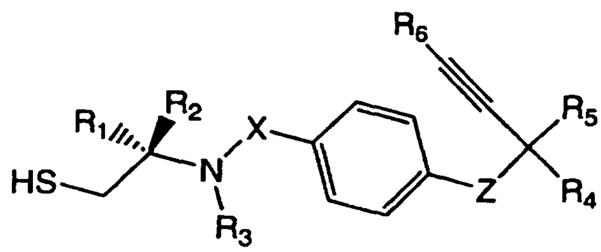

**1a**

Particularly preferred compounds of the invention are the following:

4-But-2-ynyloxy-N-((1R)-2-mercapto-1-methyl-ethyl)-N-methylbenzenesulfonamide;
(2R)-2-{[4-(2-Butynyloxy)phenyl]sulfonyl}[2-(4-morpholinyl)ethyl]amino}-3-sulfanylpropanamide, and
4-(2-Butynyloxy)-N-[(1R)-1-methyl-2-sulfanylethyl]-N-[2-(4-morpholinyl) ethyl]benzenesulfonamide.

In a further aspect, the invention relates to a compound of formula **II,** with the proviso that $R_6$ is not hydrogen and $R_4$, $R_5$ and $R_6$ are as defined in above.

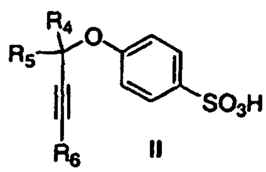

II

In a further aspect, the invention relates to a compound of formula **III,** with the proviso that $R_6$ is not hydrogen wherein J is fluorine, bromine, chlorine, 1,2,4-triazolyl, benzotriazolyl or imidazolyl, and $R_4$, $R_5$ and $R_6$ are as defined above.

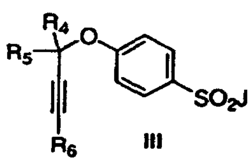

III

In a further aspect, the invention relates to the use of a compound of the invention in the preparation of a medicament for inhibiting pathological changes mediated by TNF-$\alpha$ converting enzyme (TACE) in a mammal.

In a further aspect, the invention relates to a pharmaceutical composition comprising a compound as claimed in claim 1 or a salt thereof and a pharmaceutically acceptable carrier.

## DETAILED DESCRIPTION OF THE INVENTION

The invention compounds are prepared using conventional techniques known to those skilled in the art of organic synthesis. The starting materials used in preparing the compounds of the invention are known, made by known methods or are commercially available.

Those skilled in the art will recognize that certain reactions are best carried out when other potentially reactive functionality on the molecule is masked or protected, thus avoiding undesirable side reactions and/or increasing the

yield of the reaction. To this end, those skilled in the art may use protecting groups. Examples of these protecting group moieties may be found in T. W. Greene, P. G. M. Wuts "Protective Groups in Organic Synthesis", 2nd Edition, 1991, Wiley & Sons, New York. Reactive side chain functionalities on amino acid starting materials are preferably protected. The need and choice of protecting groups for a particular reaction is known to those skilled in the art and depends on the nature of the functional group to be protected (hydroxy, amino, carboxy, etc.), the structure and stability of the molecule of which the substituent is part and the reaction conditions. Those skilled in the art will recognize that the nature and order of the synthetic steps presented may be varied for the purpose of optimizing the formation of the compounds of the invention.

When preparing or elaborating compounds of the invention containing aryl, heteroaryl or heterocyclic rings, those skilled in the art recognize that substituents on that ring may be prepared before, after or concomitant with construction of the ring. For clarity, substituents on such rings have been omitted from the schemes herein below.

The thiol compounds of the invention, **1a-1c,** are prepared according to **Scheme 1** by converting an alcohol, **2,** into the corresponding thioester or dithioester, **1b,** via a Mitsunobu procedure using reagents such as triphenylphosphine, diethyl azodicarboxylate and thiolacetic acid. Conversion of **1b** into thiol **1a** is accomplished through hydrolysis or reductive cleavage of the ester using sodium methoxide, sodium borohydride or similar reagents. Thiol **1a** may be converted into the corresponding disulfide using methods compatible with acetylenic substituents, such as oxidation with air or oxygen.

Alternatively, the alcohol moiety of **2** can be converted into a leaving group J, where J is a halide, tosylate, mesylate, triflate or similar functionality to give compound **3**. Reaction of **3** with nucleophiles such as sodium sulfide, thiolacetic acid, dithiolacetic acid, or similar agents or their salts, then provides **1a** or **1b.**

Scheme 1:

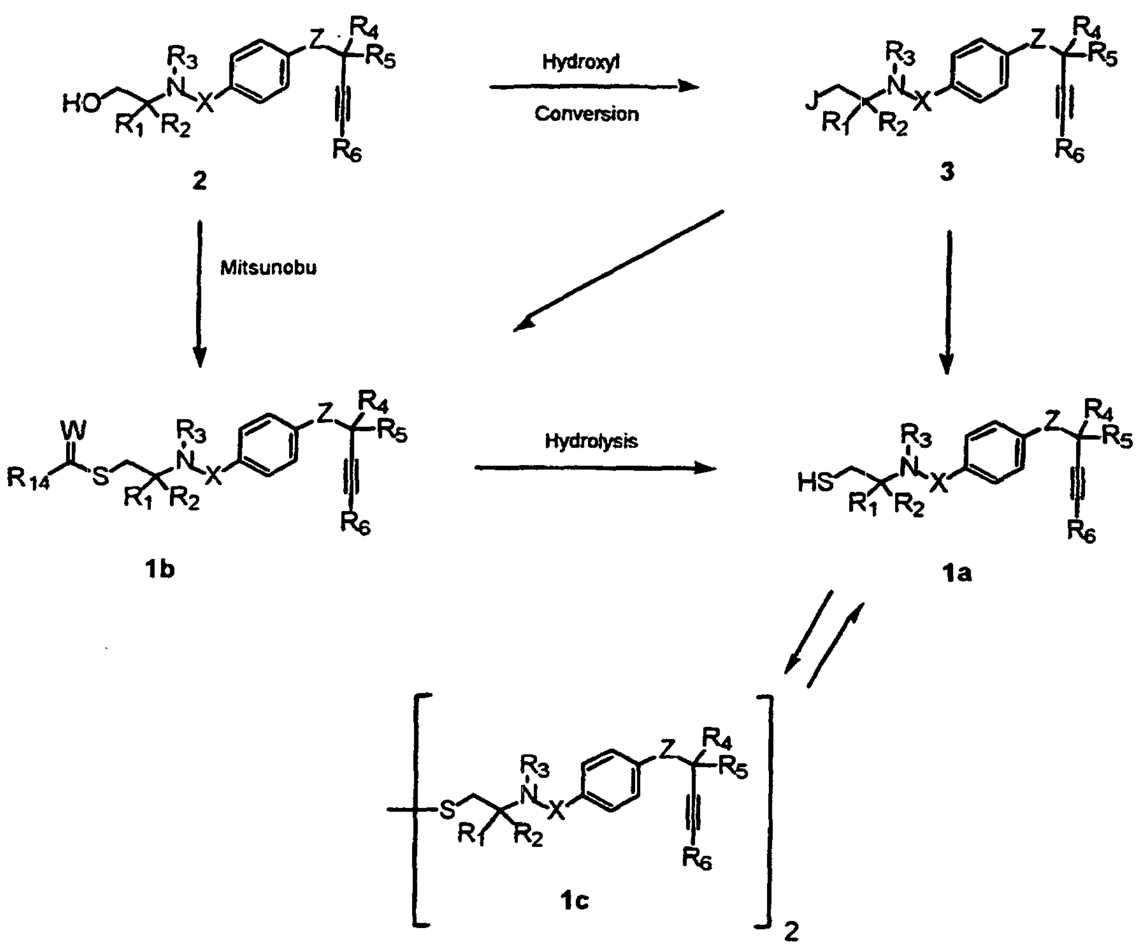

2
3
1b
1a
1c

Alcohols **2** may be prepared as shown in Scheme **2**. Amino-alcohol **4** ($R_{30}$ = H), or its hydroxyl protected analog wherein $R_{30}$ is a suitable masking group such as trialkylsilyl or tetrahydropyran can be sulfonylated or phosphorylated with **7,** wherein J is as described in **Scheme 1,** in the presence of a tertiary amine base, or pyridine, to provide 8. Alkylation of N-H compound 8 with R,J and a base such as potassium carbonate or sodium hydride in a polar aprotic solvent such as acetone, N,N-dimethylformamide (DMF), or tetrahydrofuran (THF) provides sulfonamide **9.** Compound **9** is also available through direct reaction of **7** with an N-substituted amino-alcohol derivative, **5.** Compound **5** is available via alkylation or reductive alkylation of amine **4,** or by amination of **6** or the corresponding epoxide. Conversion of **9** into the alcohol is then performed in a manner consistent with the choice of protecting group $R_{30}$ and the presence of a carbon-carbon triple bond.

Scheme 2:

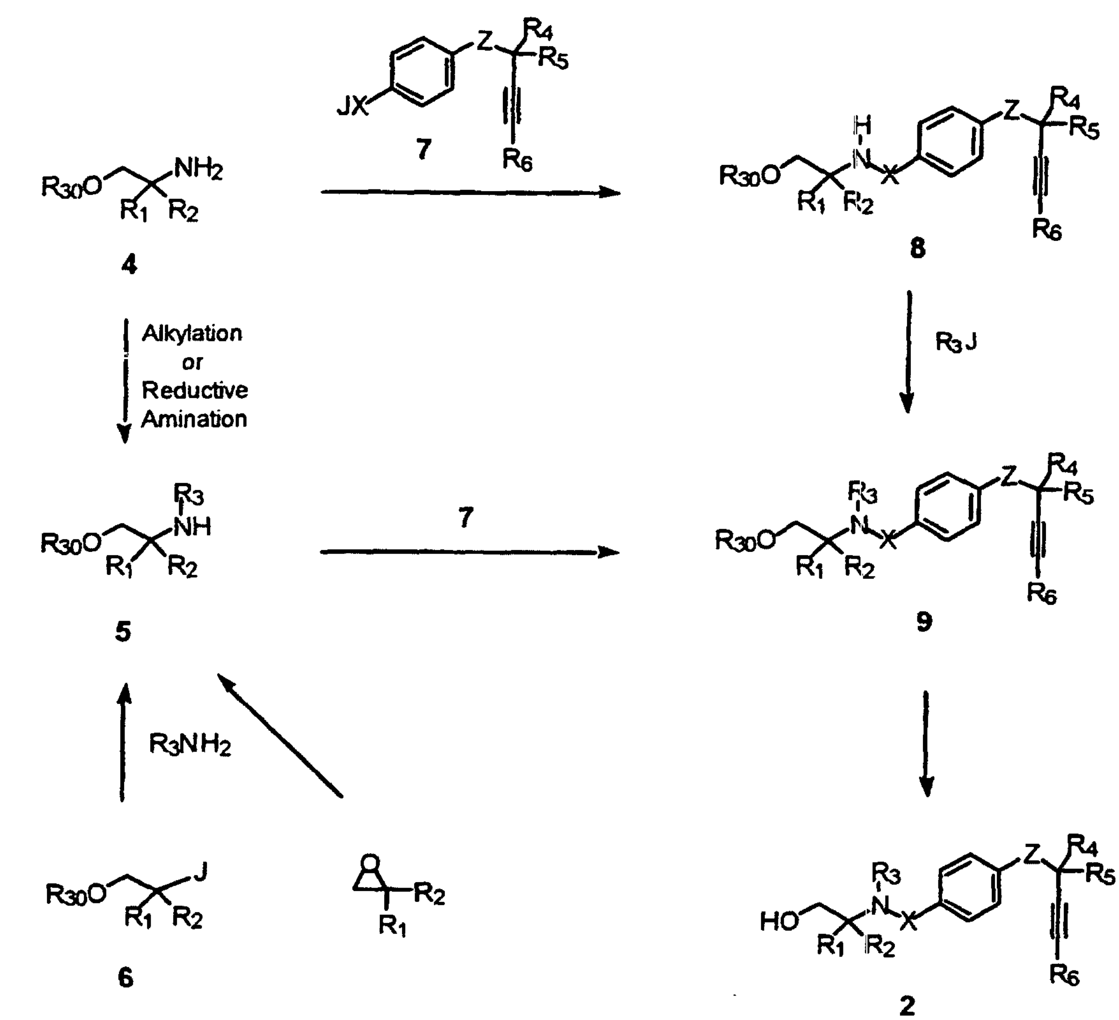


Another route to compounds **9** is shown in **Scheme 3.** Compound **7,** for example the sulfonyl chloride, can react with a primary amine or ammonia to give compounds **10** or **11,** respectively. Alkylation of **10** with **6** or an analogous epoxide provides **9** directly, whereas **11** can be alkylated with $R_3J$ followed by **6** (or vice versa) or an epoxide to give **9.**

Scheme 3:

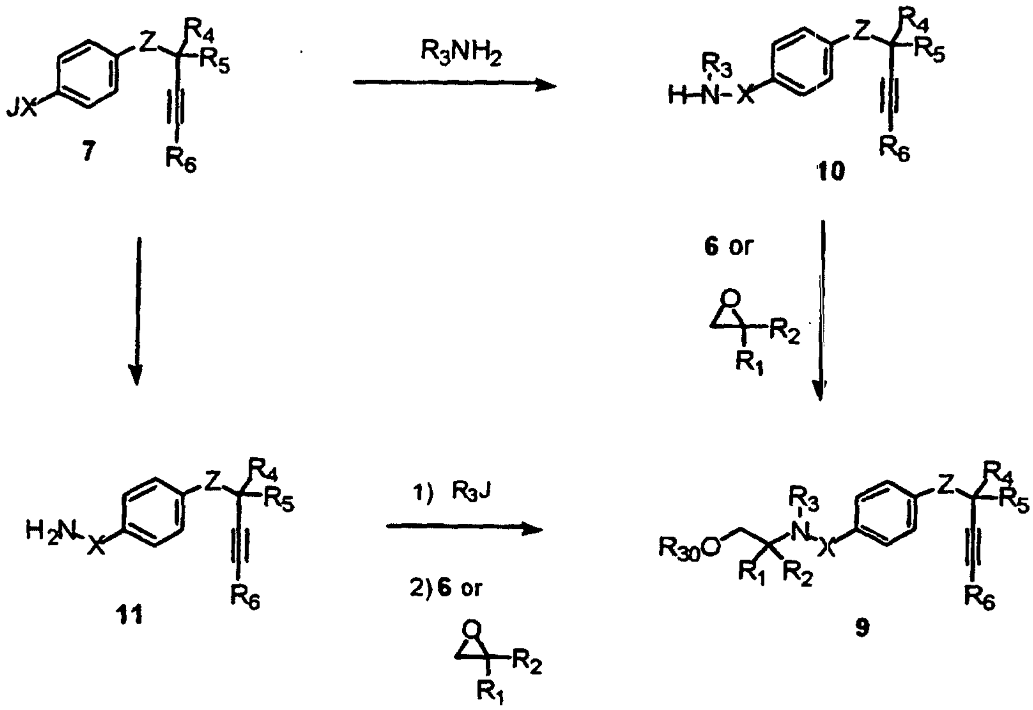


Methods of preparation of sulfonylating agents **7** are shown in **Scheme 4.** Thus, sulfonic acid salts **12,** where $ZR_{50}$ is a hydroxy, thiol or substituted amino moiety may be alkylated with acetylenes **13,** where J is a suitable leaving group such as halogen mesylate, tosylate, or triflate to give **14.** Acetylenes **13** are commercially available or known compounds, or they may be synthesized by known methods by those skilled in the art. The sulfonic acid salts **14** may be converted into the corresponding sulfonyl chloride or other sulfonylating agent **7** by known methods, such as reaction with oxalyl chloride or other reagent compatible with substituents $R_4$, $R_5$ and $R_6$ and the acetylene. Alternatively, the disulfide **15** may be converted into diacetylene **16** by reaction with compounds **13,** followed by reduction of the disulfide bond to provide the analogous thiols which may be converted into **7** by known methods. Alkylation of the phenol, thiophenol, aniline or protected aniline **17** with **13** to give **18,** followed by reaction with chlorosulfonic acid provide sulfonic acids **19** which are readily converted into **7** with oxalyl chloride or similar reagents. Thiophenols **20** are also precursors to **7** via protection of the thiol, alkylation of ZH, where Z is O, N or S, and deprotection of the sulfur followed by oxidation to the sulfonic acid **19.**

Scheme 4:

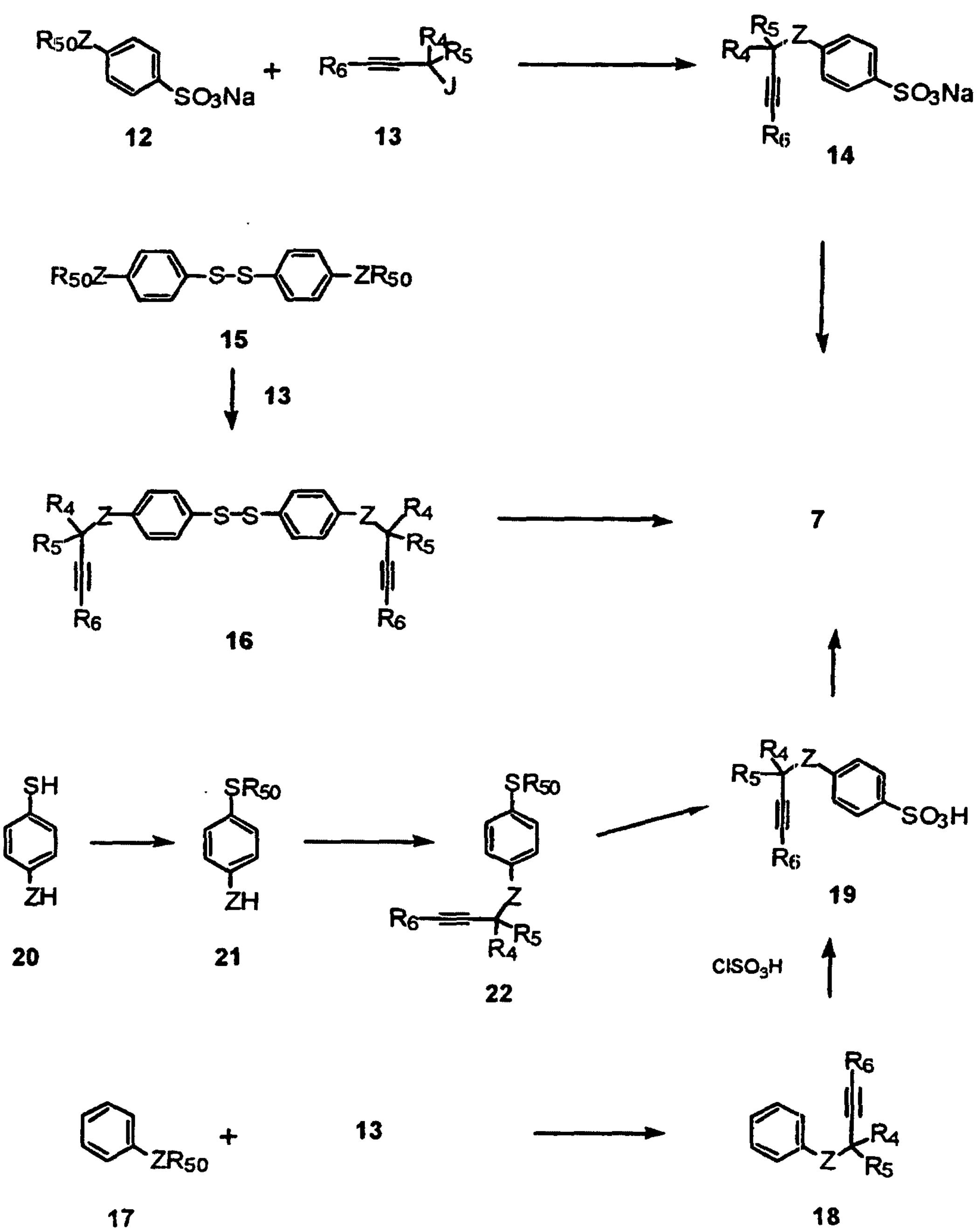


12 13 14 15 16 7 20 21 22 19 17 18

The phosphorus containing analogs of **7** may be prepared using similar methodology, as shown in **Scheme 5.**

Scheme 5:

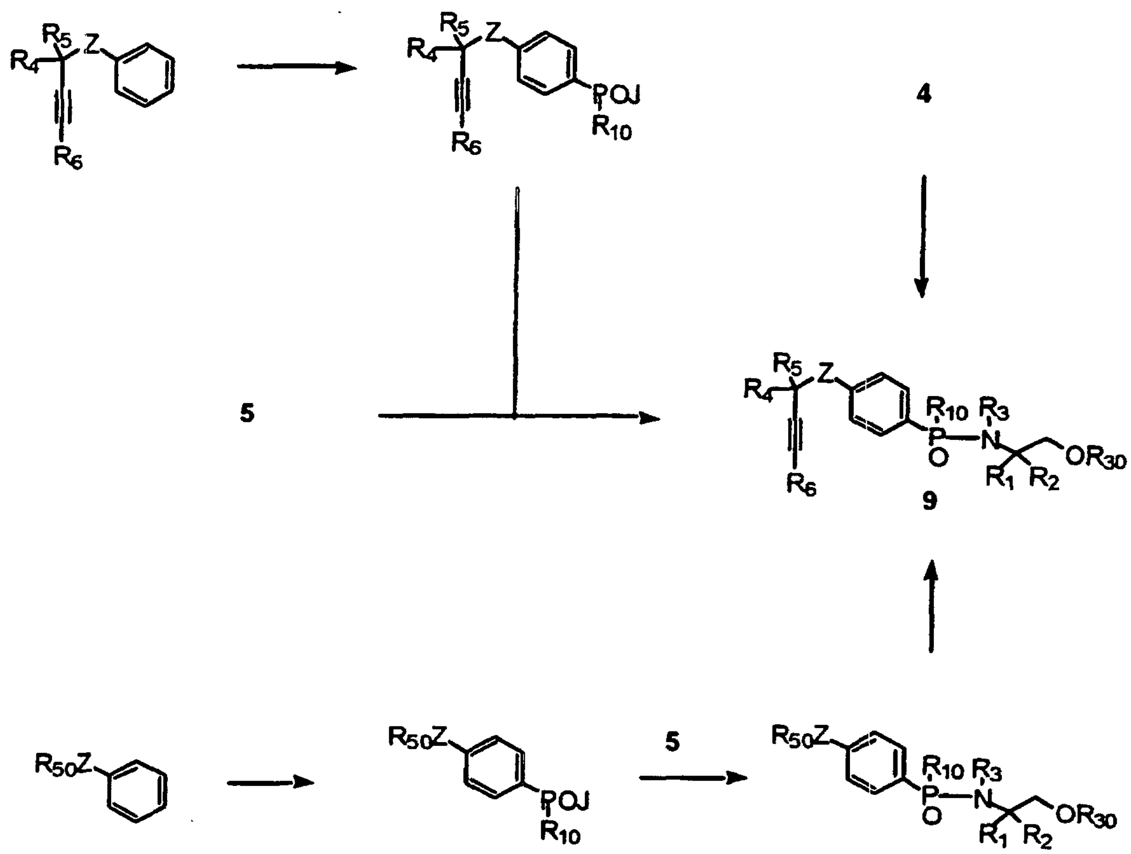


The acetylenic side chain may also be appended after sulfonylation or phosphorylation of the amino acid derivative, as shown in **Scheme 6.** Thus, the amino-alcohol derivatives **4** and **5** can be sulfonylated or phosphorylated with compounds **23,** where $ZR_{50}$ is hydroxy or protected hydroxy, thiol or amine, and, if necessary, alkylated as in **Scheme 2,** to give **24.** Removal of the $\mathbf{R_{50}}$ masking group to give **25** and subsequent alkylation of the resulting phenol, thiol or amine with **13** provides **9.** In the case where $\mathbf{ZR_{50}}$ is equal to OH, no deprotection step is required to give **25.** Alternatively, the $OR_{30}$ moiety of **24** may be converted into the analogous thioester, thiol or disulfide, as shown in **Schemes 1** and **2,** prior to deprotection of the $ZR_{50}$ moiety of compound **24.** Subsequent alkylation of the unmasked -ZH group would then provide the compounds of the invention.

Scheme 6:

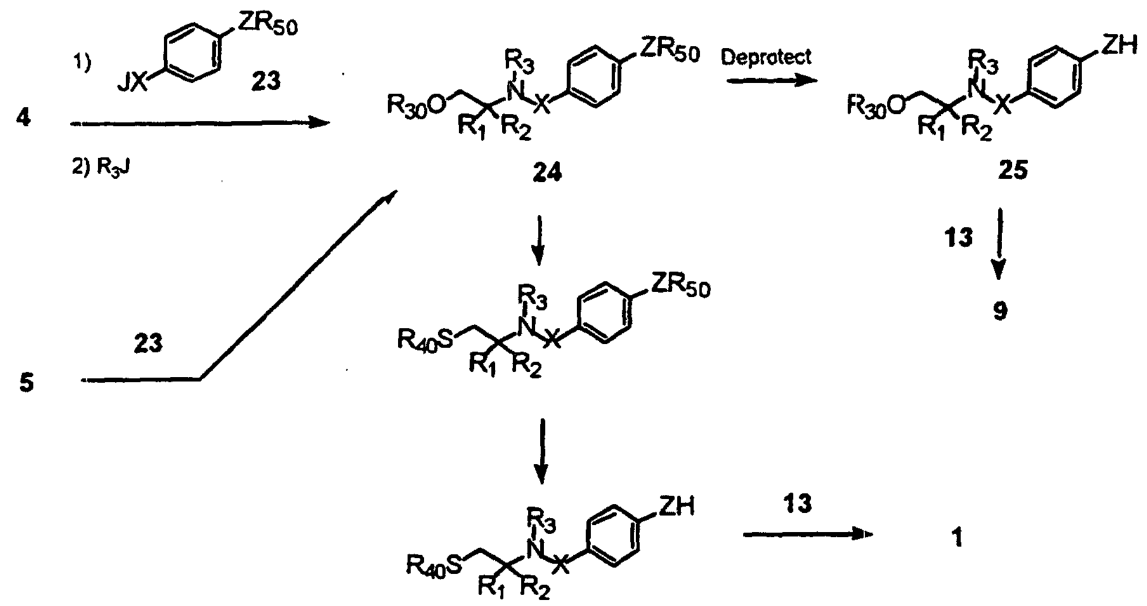


The propargylic amine analogs of 9 can be synthesized as shown in Scheme 7 starting from the amino-alcohol derivatives **4** and/or **5.** Sulfonylation or phosphorylation with para-nitro aryl compound **26,** for example 4-nitrobenzenesulfonyl chloride, followed by alkylation with $R_3J$ (for **4**) using a base such as potassium carbonate or sodium hydride in DMF provides **27.** Reduction of the nitro moiety with hydrogen and palladium on carbon, tin chloride or other known method to give aniline **28** and subsequent alkylation with **13** then provides **9.** Aniline **28** may also be derivatized **(29)** prior to alkylation with **13** and then deprotected after the alkylation step. As in **Scheme 6,** compound **29** can first be converted into its thioester, disulfide or protected thiol analog, followed by appending the propargyl group, via alkylation with **13,** and subsequent deprotection of the aniline to provide compounds **1a-1c** of the invention.

Scheme 7:

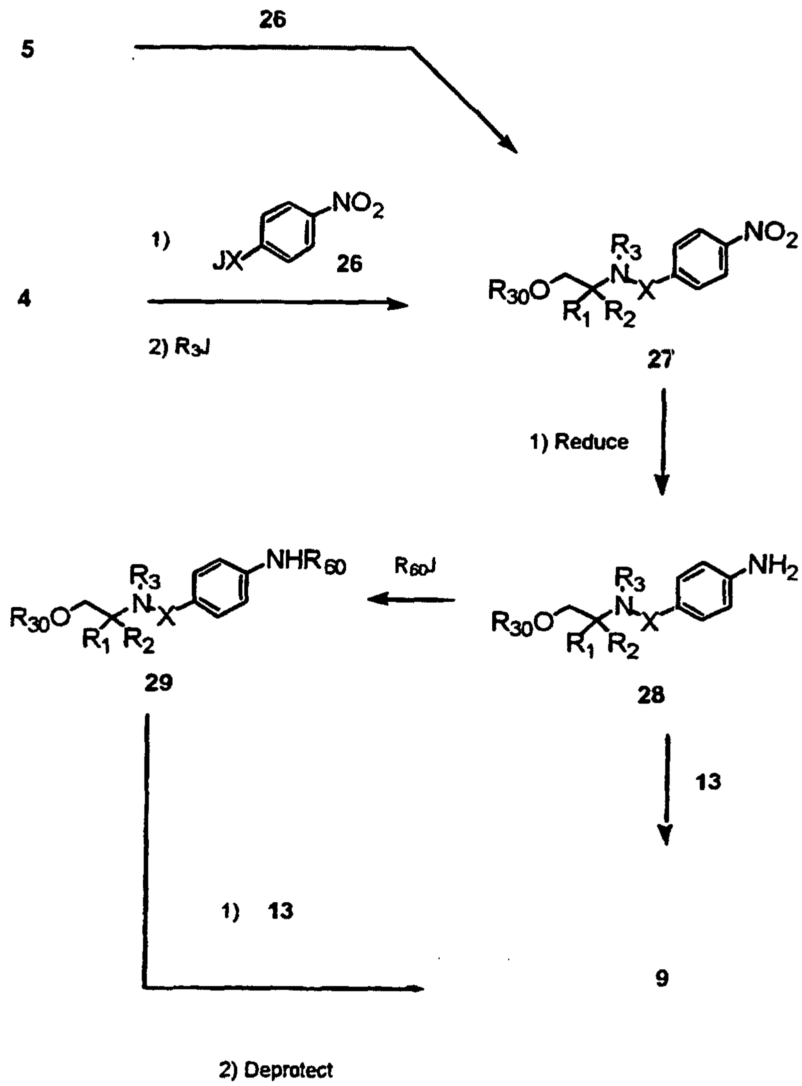


Acetylenic derivatives **9** are also accessible via the fluoro compounds **31,** readily prepared from the amino-alcohol derivatives **4** and/or **5** by reaction with fluoroaryl **30**, as shown in **Scheme 8**. Displacement of the fluorine of **31** in the presence of a base such as sodium hydride with a masked hydroxy, thiol, or amino group ($HZR_{70}$, where $R_{70}$ is a suitable protecting group) in a polar aprotic solvent such as DMF, followed by deprotection gives **32,** which can then be alkylated with **13** to provide **9.** Conversion of **31** to **32**, where Z is sulfur, might also be accomplished with $Na_2S$, $K_2S$, NaSH or KS(C=S)OEt. The fluorine of **31** can also be displaced in a polar aprotic solvent with the propargylic derivative **33,** where Z is O, S or NH, in the presence of a base such as sodium hydride, to give **9** directly. As described for Schemes **6** and **7** the order of synthetic operations may be changed such that the acetylcnic moiety is appended after conversion of $OR_{30}$ into the corresponding thioester, disulfide or protected thiol.

Scheme 8:

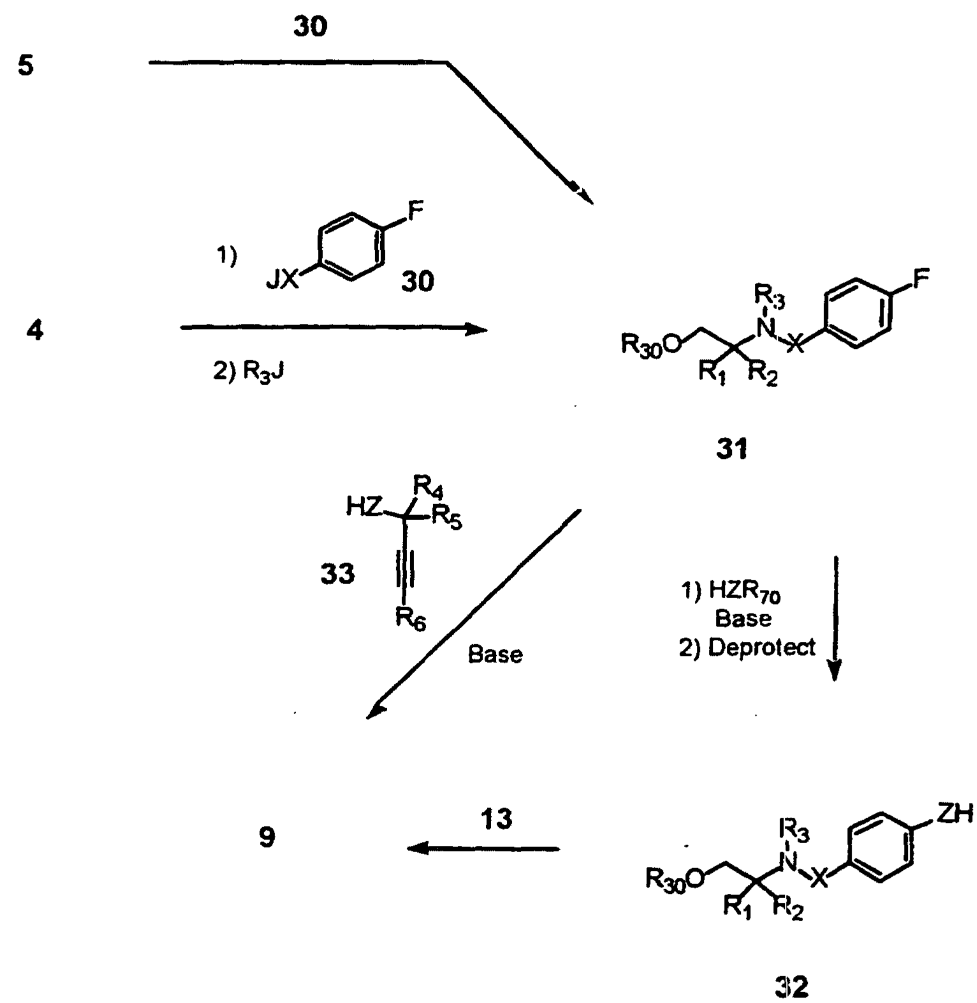


Compound **9,** wherein Z is a methylene group, is available via **34,** as shown in **Scheme 9.** Benzylic bromination of **34** with N-bromosuccinimide in a chlorinated hydrocarbon solvent provides bromide **35.** This is followed by displacement of the bromide with the appropriate propynyl cuprate to provide sulfonamide 9.

Scheme 9:

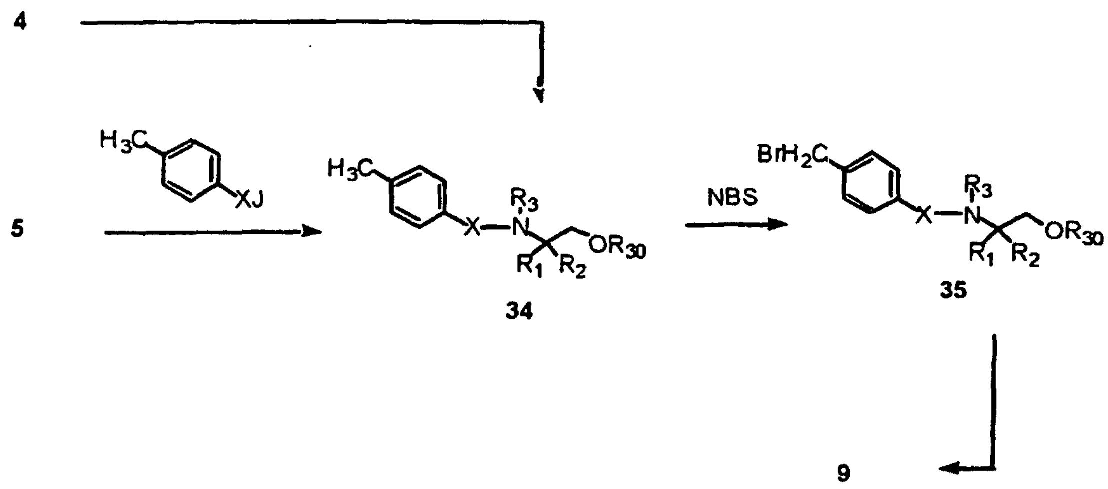


4

5

34

35

9

Some of the methods available for the derivatization of compounds of structure **9** (for the case wherein $R_6$ is hydrogen) are shown in **Scheme 10.** Metallation of the terminal acetylene **9** followed by addition of an aldehyde or alkyl halide, sulfonate or triflate provides derivatives **36** and **37.** Reaction of **9** with formaldehyde and an amine provides the Mannich addition product **38.** Cyanogen bromide addition to **38** gives the propargylic bromide **39** which may be displaced with a variety of nucleophiles to give, for example, ethers, thioethers and amines, **40.** Palladium catalyzed coupling reactions of **9** provide the aryl or heteroaryl acetylenes **41.** It is recognized by those skilled in the art of organic synthesis that the successful use of these methods is dependent upon the compatibility of substituents on other parts of the molecule. Protecting groups and/or changes in the order of steps described herein may be required. Examples of $R_{35}$, $R_{45}$, $R_{55}$, $R_{65}$ and $R_{75}$ are alkyl groups such as methyl.

Scheme 10:

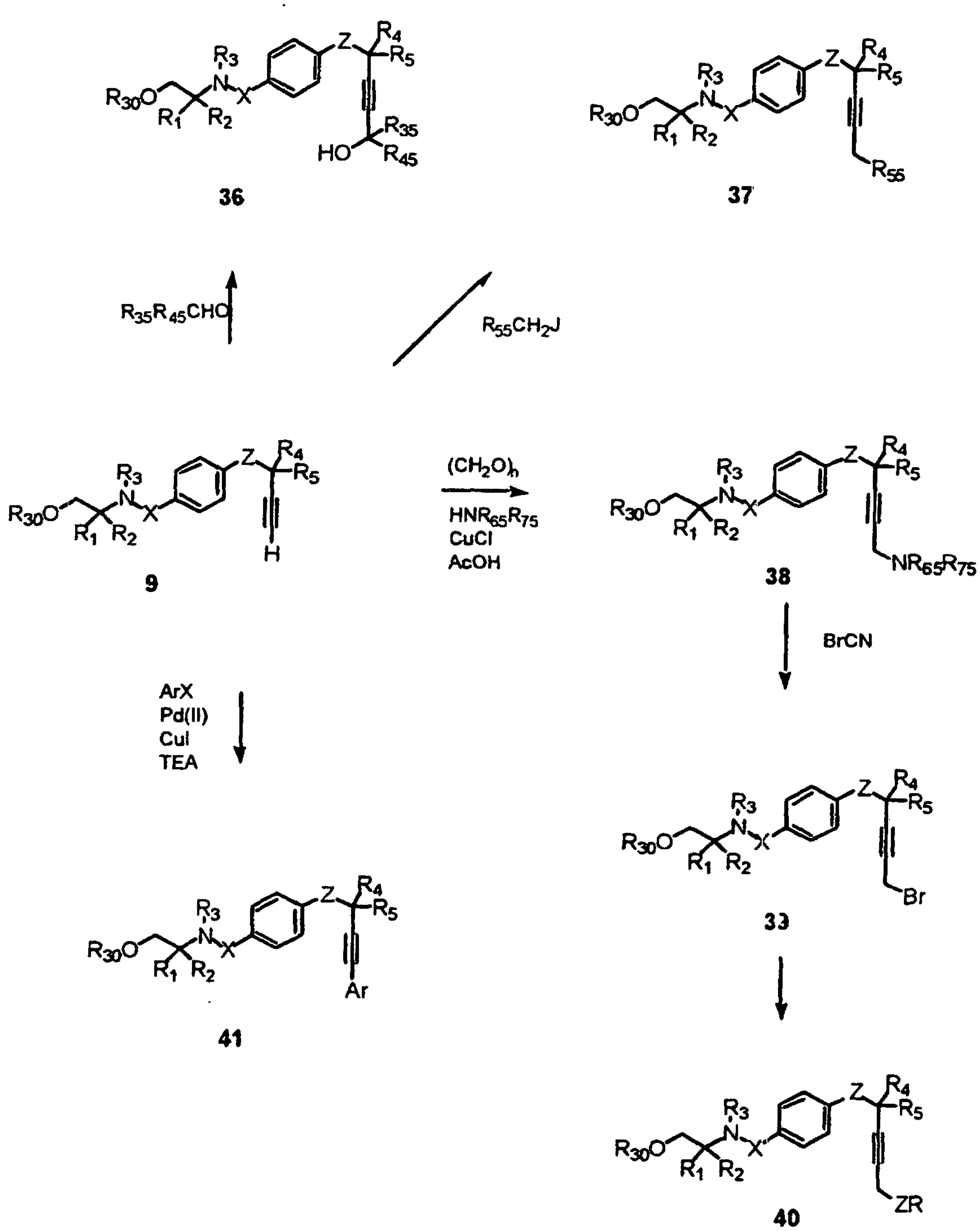


The following specific examples illustrate the preparation of representative compounds of this invention. The starting materials, intermediates, and reagents are either commercially available or can be readily prepared following standard literature procedures by one skilled in the art of organic synthesis.

### Example 1

**4-But-2-ynyloxy-benzenesulfonic acid sodium salt**

To a solution of 52.35g (0.225 mol) of 4-hydroxybenzenesulfonate sodium salt in 1L of isopropanol and 225 mL of a 1.0N solution of sodium hydroxide was added 59.96g (0.45 mol) of 1-bromo-2-butyne. The resulting mixture was heated to 70° for 15h and then the isopropanol was removed by evaporation in vacuo. The resulting white precipitate was collected by filtration, washed with isopropanol and ether and dried in vacuo to give 56.0g (100%) of the butynyl ether as a white solid.

### Example 2

**4-But-2-ynyloxy-benzenesulfonyl chloride**

To a 0° solution of 43.8 mL (0.087 mol) of 2M oxalyl chloride/dichloromethane solution in 29 mL of dichloromethane was dropwise added 6.77 mL (0.087 mol) of DMF followed by 7.24g (0.029 mol) of the product of **Example 1.** The reaction mixture was stirred for 10 minutes at 0° then let warm to room temperature and stirred for 2 days. The reaction was then poured into ice and extracted with 150 mL of hexanes. The organics were washed with water and brine, dried over $Na_2SO_4$, filtered and concentrated in vacuo to provide 6.23g (88%) of the sulfonyl chloride as a yellow solid; m.p. 63-65°C. EI Mass Spec: 243.9 ($M^+$).

### Example 3

**But-2-ynyloxy-benzene**

To a solution of 6.14g (0.023 mol) of triphenylphosphine dissolved in 100 mL of benzene and 40 mL of THF was added 1.75 mL (0.023 mol) of 2-butyn-1-ol. After five minutes 2.00 (0.023 mol) phenol, dissolved in 10 mL of THF, was added to the reaction followed by 3.69 mL (0.023 mol) of diethyl azodicarboxylate. The resulting reaction mixture was stirred for 18h at room temperature and then concentrated in vacuo. The residue was chromatographed on silica gel eluting with ethyl acetate/hexanes (1:10) to provide 2.18g (70%) of the butynyl ether as a clear liquid. EI Mass Spec: 146.0 MH+

### Example 4

**4-But-2-ynyloxy-benzenesulfonyl chloride**

To a solution of 0.146g (1.0 mmol) of the product of **Example 3** in 0.3 mL of dichloromethanein an acetone/ice bath under $N_2$ was dropwise added a solution of 0.073 mL (1.1 mmol) of chlorosulfonic acid in 0.3 mL of dichloromethane. After the addition was complete, the ice bath was removed and the reaction was stirred at room temperature for 2h. To the reaction was then dropwise added 0.113 mL (1.3 mmol) of oxalyl chloride, followed by 0.015 mL DMF. The reaction was heated to reflux for 2h and then diluted with hexane and poured into ice water. The organic layer was washed with brine, dried over sodium sulfate, and concentrated in vacuo to provide 0.130mg (53%) of the desired product as a light brown solid.

### Example 5

**4-But-2-ynyloxy-N-(2-hydroxy-1-methyl-ethyl)-benzenesulfonamide**

To a solution of 0.279g (3.718 mmol) of (*R*) -(-)-2-amino-1-propanol in 2.6 mL of THF and 0.9 mL of water was added 0.62 mL of triethylamine followed by 1.00g (4.09 mmol) of 4-but-2-ynyloxy-benzenesulfonyl chloride and the resulting mixture was stirred at room temperature for 15h. The reaction was then diluted with ethyl acetate and washed with 5% HCl solution and water, dried over $MgSO_4$, filtered and concentrated in vacuo. The resulting solid was washed with ether and dried in vacuo to provide 0.873g (83%) of the sulfonamide as a white solid. Electrospray Mass Spec: 283.8 $(M+H)^+$

**Example 6**

**4-But-2-ynyloxy-N-(2-hydroxy-1-methyl-ethyl)-N-methyl-benzenesulfonamide**

To a solution of 0.400g (1.413 mmol) of the product of **Example 5** in 3.0 mL of DMF was added 0.585g (4.240 mmol) of potassium carbonate followed by 0.132 mL (2.12 mmol) of iodomethane and the resulting mixture was stirred at room temperature for 12h. The reaction was then diluted with ether and washed with water, dried over $MgSO_4$, filtered and concentrated in vacuo to provide 0.354g (84%) of the N-methyl sulfonamide as a white solid. Electrospray Mass Spec: 297.9 $(M+H)^+$

**Example 7**

**Thioacetic acid S-{2-[(4-but-2-ynyloxy-benzenesulfonyl)-methyl-amino]-propyl}ester**

To a 0°C solution of 0.302g (1.017 mmol) of the product of **Example 6** and 0.293g (1.118 mmol) of triphenylphosphine in 4.0 mL of THF was added 0.176 mL (1.118 mmol) of diethyl azodicarboxylate. The resulting mixture was stirred for 0.5h at 0°C and then concentrated in vacuo. The residue was chromatographed on silica gel eluting with ethyl acetate/hexanes to provide 0.228g (63%) of the thioacetate as a colorless oil. Electrospray Mass Spec: 355.9 $(M+H)^+$

**Example 8**

**4-But-2-ynyloxy-N-((1R)-2-mercapto-1-methyl-ethyl)-N-methyl-benzenesulfonamide**

To a solution of 0.168g (0.473 mmol) of the product of **Example 7** in 2.1 mL of methanol was added 0.092g (1.704 mmol) of sodium methoxide. After stirring at room temperature for 2h the reaction was quenched with 5% HCl solution and extracted with ether. The organics were dried opver Na2SO4, filtered and concentrated in vacuo. The residue was chromatographed on silica gel eluting with ethyl acetate/hexanes in a gradient from (1:10) to (1:3) to provide 0.148g (100%) of the thiol as a colorless oil. Electrospray Mass Spec: 313.9 $(M+H)^+$

**Example 9**

**4-But-2-ynyloxy-N-(2-hydroxy-1-methyl-ethyl)-N-(2-morpholin-4-yl-ethyl)-benzenesulfonamide**

According to the procedure of Example 6, 0.400g (1.413 mmol) of the product of **Example 5** and 0.289g (1.555 mmol) of 4-(2-chloroethyl)morpholine hydrochloride provided 0.334g (60%) of the N-morpholinoethyl sulfonamide as a colorless oil. Electrospray Mass Spec: 397.0 $(M+H)^+$

**Example 10**

**S-((2R)-2-{{[4-(2-Butynyloxy)phenyl]sulfonyl}[2-(4-morpholinyl)ethyl]amino}propyl)ethanethioate**

To a solution of 0.416g (1.16 mmol) of the product of **Example 9** in 4 mL of THF were added sequentially 0.304g (1.16 mmol) of triphenylphosphine, 0.191g (1.16 mmol) of diethyl azodicarboxylate and 0.25 mL (3.5 mmol) of thiolacetic acid. The reaction was stirred for 45h, reduced to dryness and the resulting residue was subjected to flash chromatography eluting with ethyl acetate/ hexanes (3:1) to provide 0.5g (95%) of the thioacetate as a white solid. Electrospray Mass Spec: 455.3 $(M+H)^+$

**Example 11**

**4-(2-Butynyloxy)-N-[(1R)-1-methyl-2-sulfanylethyl]-N-[2-(4-morpholinyl)ethyl]benzenesulfonamide**

To a solution of0.16g (0.352 mmol) of the product of **Example 10** in 2 mL of methanol in a sealable tube at -78° was added 20 mL of liquid ammonia. The tube was sealed and the reaction stirred for 12h at room temperature. After recooling to -78° the reaction tube was unsealed and the solution was carefully reduced to dryness. The residue was chromatographed on silica gel eluting with methylene chloride/methanol (50:1) furnishing 121mg (84%) of the desired thiol as a white solid. Electrospray Mass Spec: 413.4 $(M+H)^+$

**Example 12**

**(2R)-2-Amino-3-(tritylsulfanyl)propanamide**

To 2.68g (7.37 mmol) of S-trityl-L-cysteine and 40 mL of methanol was added 7 mL (96 mmol) of thionyl chloride dropwise. After heating at reflux for 6h the solution was cooled to room temperature and then concentrated in vacuo. The resulting residue was taken up in 20 mL of methanol, treated with activated carbon, filtered and concentrated in vacuo yielding the methyl ester as an off-white foam. This material was dissolved in 6 mL of methanol in a sealable tube and cooled to -78°. After 30 mL of liquid ammonia was added, the tube was sealed and the reaction was stirred at room temperature for 14h After recooling to -78° the reaction tube was unsealed and the solution was carefully reduced to dryness. The residue was chromatographed on silica gel eluting with methylene chloride/methanol (10:1) furnishing 1.52g (57%) of the primary amide as a white solid. Electrospray Mass Spec: 363.2 $(M+H)^+$

**Example 13**

**(2R)-2-({[14-(2-Butynyloxy)phenyl]sulfonyl}amino)-3-(tritylsulfanyl)propanamide**

To a solution of 1.203g (3.685 mmol) of the primary amide product from **Example 12** and 1.39 mL (10 mmol) of triethylamine in 20 mL of methylene chloride was added in one portion 0.954g (3.9 mmol) of 4-but-2-ynyloxy-benzenesulfonyl chloride. After stirring for 13h, 50 mL of dichloromethane and 50 mL of water were added. The organic layer was washed with brine, dried over sodium sulfate, filtered, concentrated in vacuo and subjected to flash chromatography eluting with hexanes/ethyl acetate (1:1) to furnish 1.65g (79%) of the desired sulfonamide as a white solid. Electrospray Mass Spec: 1139.6 (2M-H)

**Example 14**

**(2R)-2-{{[4-(2-Butynyloxy)phenyl]sulfonyl}[2-(4-morpholinyl)ethyl]amino}-3-(tritylsulfanyl)propanamide**

To a solution of 0.6482g (1.136 mmol) of the product from Example 13 in 3 mL of DMF was added 0.317g (1.704 mmol) of 4-(2-chloroethyl) morpholine hydrochloride and 0.705g (5.1 mmol) of potassium carbonate. The resulting mixture was heated at 60° for 14h. After cooling to room temperature the reaction mixture was diluted with ethyl acetate and washed with water. The organic layer was dried over sodium sulfate, filtered, concentrated in vacuo and subjected to flash chromatography eluting with hexanes/ethyl acetate (1:5) to furnish 0.434g (56%) of the desired compound as a white solid. Electrospray Mass Spec: 684.5 $(M+H)^+$

**Example 15**

**(2R)-2-{{[4-(2-Butynyloxy)phenyl]sulfonyl}[2-(4-morpholinyl)ethyl]amino}-3-sulfanylpropanamide**

To a solution of 0.116g (0.170 mmol) of the product from **Example 14** in 1.5 mL of methylene chloride was added triisopropylsilane, followed by trifluoroacetic acid. Upon consumption of starting material the solution was concentrated in vacuo and washed four times with 2mL of ether. The residue was partitioned between ethyl acetate and saturated aqueous sodium bicarbonate. The organic layer was dried over sodium sulfate, filtered and concentrated in vacuo furnishing 66 mg (88%) of the thiol as a white solid. Electrospray Mass Spec: 442.4 $(M+H)^+$

Pharmacology

The ability of the compounds of the invention, or their pharmaceutically acceptable salts, to inhibit matrix metalloproteinases or TACE and, consequently, demonstrate their effectiveness for treating diseases modulated by matrix metalloproteinases or TACE is shown by the following in vitro assays.

Test Procedures for Measuring MMP-1, MMP-9, and MMP-1:3 Inhibition

These standard pharmacological test procedures are based on the cleavage of a thiopeptide substrates such as Ac-Pro-Leu-Gly(2-mercapto-4-methyl-pentanoyl)-Leu-Gly-OEt by the matrix metalloproteinases MMP-1, MMP-13 (collagenases) or MMP-9 (gelatinase), which results in the release of a substrate product that reacts colorimetrically with DTNB (5,5'-dithiobis(2-nitro-benzoic acid)). The enzyme activity is measured by the rate of the color increase. The thiopeptide substrate is made up fresh as a 20 mM stock in 100% DMSO and the DTNB is dissolved in 100%

DMSO as a 100 mM stock and stored in the dark at room temperature. Both the substrate and DTNB are diluted together to 1 mM with substrate buffer (50 mM HEPES pH 7.5, 5 mM $CaCl_2$) before use. The stock of enzyme is diluted with buffer (50 mM HEPES, pH 7.5, 5 mM $CaCl_2$, 0.02% Brij) to the desired final concentration. The buffer, enzyme, vehicle or inhibitor, and DTNB/substrate are added in this order to a 96 well plate (total reaction volume of 200 μl) and the increase in color is monitored spectrophotometrically for 5 minutes at 405 nm on a plate reader and the increase in color over time is plotted as a linear line.

Alternatively, a fluorescent peptide substrate is used. In this test procedure, the peptide substrate contains a fluorescent group and a quenching group. Upon cleavage of the substrate by an MMP, the fluorescence that is generated is quantitated on the fluorescence plate reader. The assay is run in HCBC assay buffer (50mM HEPES, pH 7.0, 5 mM $Ca^{+2}$, 0.02% Brij, 0.5% Cysteine), with human recombinant MMP-1, MMP-9, or MMP-13. The substrate is dissolved in methanol and stored frozen in 1 mM aliquots. For the assay, substrate and enzymes are diluted in HCBC buffer to the desired concentrations. Compounds are added to the 96 well plate containing enzyme and the reaction is started by the addition of substrate. The reaction is read (excitation 340 nm, emission 444 nm) for 10 min. and the increase in fluorescence over time is plotted as a linear line.

For either the thiopeptide or fluorescent peptide test procedures, the slope of the line is calculated and represents the reaction rate. The linearity of the reaction rate is confirmed ($r^2$ >0.85). The mean (x±sem) of the control rate is calculated and compared for statistical significance (p<0.05) with drug-treated rates using Dunnett's multiple comparison test. Dose-response relationships can be generated using multiple doses of drug and $IC_{50}$ values with 95% CI are estimated using linear regression.

Test Procedure for Measuring TACE Inhibition

Using 96-well black microtiter plates, each well receives a solution composed of 10 μL TACE (final concentration 1μg/mL), 70μL Tris buffer, pH 7.4 containing 10% glycerol (final concentration 10 mM), and 10 μL of test compound solution in DMSO (final concentration 1μM, DMSO concentration <1%) and incubated for 10 minutes at room temperature. The reaction is initiated by addition of a fluorescent peptidyl substrate (final concentration 100 μM) to each well and then shaking on a shaker for 5 sec.

The reaction is read (excitation 340 nm, emission 420 nm) for 10 min. and the increase in fluorescence over time is plotted as a linear line. The slope of the line is calculated and represents the reaction rate.

The linearity of the reaction rate is confirmed ($r^2$ >0.85). The mean (x±sem) of the control rate is calculated and compared for statistical significance (p<0.05) with drug-treated rates using Dunnett's multiple comparison test. Dose-response relationships can be generate using multiple doses of drug and $IC_{50}$ values with 95% CI are estimated using linear regression.

Human Monocytic THP-1 Cell Differentiation Assay For Soluble Proteins (THP-1 Soluble Protein Assay)

Mitogenic stimulation of THP-1 cells cause differentiation into macrophage like cells with concomitant secretion of tumor necrosis factor (TNF-α) and TNF receptor (TNF-R p75/80 and TNF-R p55/60) and Interleukin-8 (IL-8), among other proteins. In addition, non-stimulated THP-1 cells shed both the p75/80 and the p55/64 receptors over time. The release of membrane bound TNF-α and possibly TNF-R p75/80 and TNF-R p55/60, but not IL-8, is mediated by an enzyme called TNF-α converting enzyme or TACE. This assay can be used to demonstrate either an inhibitory or a stimulatory compound effect on this TACE enzyme and any cytotoxic consequence of such a compound.

THP-1 cells (from ATCC) are a human monocytic cell line which were obtained from the peripheral blood of a one year old male with acute monocytic leukemia. They can be grown in culture and differentiated into macrophage like cells by stimulation with mitogens.

For the assay, THP-1 cells are seeded from an ATCC stock which was previously grown and frozen back at 5 x 106/ml/vial. One vial is seeded into a T25-flask with 16 mls of RPMI-1640 with glutamax (Gibco) media containing 10 % fetal bovine serum, 100 units/ml penicillin, 100 μg/ml streptomycin, and 5 x $10^{-5}$ M 2-mercapto-ethanol (THP-1 media). Each vial of cells are cultured for about two weeks prior to being used for an assay and then are used for only 4 to 6 weeks to screen compounds. Cells are subcultured on Mondays and Thursdays to a concentration of 1 x 105/ml.

To perform an assay, the THP-1 cells are co-incubated in a 24 well plate with 50 ml/well of a 24 mg/ml stock of Lipopolysacharide (LPS) (Calbiochem Lot# B13189) at 37°C in 5% $CO_2$ at a concentration of 1.091 x $10^6$ cells/ml (1.1 ml/well) for a total of 24 hours. At the same time, 50 ml/well of drug, vehicle or THP-1 media is plated in appropriate wells to give a final volume of 1.2 ml/well. Standard and test compounds are dissolved in DMSO at a concentration of 36 mM and diluted from here to the appropriate concentrations in THP-1 media and added to the wells at the beginning of the incubation period to give final concentrations of 100 mM, 30 mM, 10 mM, 3 mM, I mM, 300 nM, and 100 nM. Cell exposure to DMSO was limited to 0.1 % final concentration. Positive control wells were included in the experiment which had mitogen added but no drug. Vehicle control wells were included as well, which were identical to the positive

control wells, except that DMSO was added to give a final concentration of 0.083%. Negative control wells were included in the experiment which had vehicle but no mitogen or drug added to the cells. Compounds can be evaluated for their effect on basal (non-stimulated) shedding of the receptors by replacing the LPS with 50 ml/well of THP-1 media. Plates are placed into an incubator set at 5% CO2 and at 37°C. After 4 hours of incubation, 300 ml/well of tissue culture supernatant (TCS) is removed for use in an TNF-α ELISA. Following 24 hours of incubation, 700 ml/well of TCS is removed and used for analysis in TNF-R p75/80, TNF-R p55/60 and IL-8 ELISAs.

In addition, at the 24 hours timepoint, and the cells for each treatment group are collected by resuspension in 500 μl/well of THP-1 media and transferred into a FACS tube. Two ml/tube of a 0.5 mg/ml stock of propidium iodide (PI) (Boerhinger Mannheim cat. # 1348639) is added. The samples are run on a Becton Dickinson FaxCaliber FLOW cytometry machine and the amount of dye taken up by each cell is measured in the high red wavelength (FL3). Only cells with compromised membranes (dead or dying) can take up PI. The percent of live cells is calculated by the number of cells not stained with PI, divided by the total number of cells in the sample. The viability values calculated for the drug treated groups were compared to the viability value calculated for the vehicle treated mitogen stimulated group ("vehicle positive control") to determine the "percent change from control". This "percent change from control" value is an indicator of drug toxicity.

The quantity of soluble TNF-α, TNF-R p75/80 and TNF-R p55/60 and IL-8 in the TCS of the THP-1 cell cultures are obtained with commercially available ELISAs from R&D Systems, by extrapolation from a standard curve generated with kit standards. The number of cells that either take up or exclude PI are measured by the FLOW cytometry machine and visualized by histograms using commercially available Cytologic software for each treatment group including all controls.

Biological variability in the magnitude of the response of THP-1 cell cultures requires that experiments be compared on the basis of percent change from "vehicle positive control" for each drug concentration. Percent change in each soluble protein evaluated from the "vehicle positive control" was calculated for each compound concentration with the following formula:

$$\%\ \text{Change} = \frac{\text{pg/ml (compound) - pg/ml (veh pos control)}}{\text{pg/ml (veh pos control) - pg/ml (veh neg control)}} \times 100$$

For the soluble protein (TNF-α, p75/80, p55/60, IL-8) studies under stimulated conditions, the mean pg/ml of duplicate wells were determined and the results expressed as percent change from "vehicle positive control". For the soluble protein (p75/80 and p55/60 receptors) studies under non-stimulated conditions, the mean pg/ml of duplicate wells were determined and the results expressed as percent change from "vehicle positive control" utilizing the following formula:

$$\%\ \text{Change} = \frac{\text{pg/ml (compound neg control) - pg/ml (veh neg control}}{\text{pg/ml (veh neg control)}} \text{X } 100$$

$IC_{50}$ values for each compound are calculated by non-linear regression analysis using customized software utilizing the JUMP statistical package.

For the cell viability studies, the viabilities (PI exclusion) of pooled duplicate wells were determined and the results expressed as % change from "vehicle positive control". The viability values calculated for the compound treated groups were compared to the viability value calculated for the "vehicle positive control" to determine "percent change from control" as below. This value "percent change from control" is an indicator of drug toxicity.

$$\%\ \text{Change} = \frac{\text{\% live cells (compound)}}{\text{\% live cells (veh pos control)}} -1 \text{ X } 100$$

References:

Bjomberg, F., Lantz, M., Olsson, I., and Gullberg, U. Mechanisms involved in the processing of the p55 and the p75 tumor necrosis factor (TNF) receptors to soluble receptor forms. Lymphokine Cytokine Res. 13:203-211, 1994.

Gatanaga, T., Hwang, C., Gatanaga, M., Cappuccini, F., Yamamoto, R., and Granger, G. The regulation of TNF mRNA synthesis, membrane expression, and release by PMA- and LPS-stimulated human monocytic THP-1 cells in vitro. Cellular Immun. 138:1-10, 1991.

Tsuchiya, S., Yamabe, M., Yamaghughi, Y., Kobayashi, Y., Konno, T., and Tada, K. Establishment and characterization of a human acute monocytic leukemia cell line (THP-1 ). Int. J. Cancer. 26:1711-176, 1980.

Results of the above in vitro matrix metalloproteinase inhibition, TACE inhibition and THP standard pharmacological test procedures are given in Table 1 below.

**Table 1.**

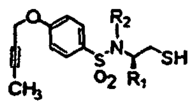

| Example # | $R_1$ | $R_2$ | MMP-1[a] | MMP-9[a] | MMP-13[a] | TACE[a] | THP |
|---|---|---|---|---|---|---|---|
| 8 | $CH_3$ | $CH_3$ | 6,300 | 2,700 | 679 | 273 | 3 |
| 11 | $CH_3$ | $(CH_2)_2$Morph | - | - | - | 362 | 11 |
| 15 | $CONH_2$ | $(CH_2)_2$Morph | - | - | - | 263 | 19 |

a) $IC_{50}$ (nM)

Based on the standard pharmacological test procedures described above, the compounds of this invention are useful in the treatment of disorders such as arthritis, tumor metastasis, tissue ulceration, abnormal wound healing, periodontal disease, graft rejection, insulin resistance, bone disease and HIV infection.

The compounds of this invention are also useful in treating or inhibiting pathological changes mediated by matrix metalloproteinases such as atherosclerosis, atherosclerotic plaque formation, reduction of coronary thrombosis from atherosclerotic plaque rupture, restenosis, MMP-mediated osteopenias, inflammatory diseases of the central nervous system, skin aging, angiogenesis, tumor metastasis, tumor growth, osteoarthritis, rheumatoid arthritis, septic arthritis, corneal ulceration, proteinuria, aneurysmal aortic disease, degenerative cartilage loss following traumatic joint injury, demyelinating diseases of the nervous system, cirrhosis of the liver, glomerular disease of the kidney, premature rupture of fetal membranes, infammatory bowel disease, age related macular degeneration, diabetic retinopathy, proliferative vitreoretinopathy, retinopathy of prematurity, ocular inflammation, keratoconus, Sjogren's syndrome, myopia, ocular tumors, ocular angiogenesis/neovascularization and corneal graft rejection.

Compounds of this invention may be administered neat or with a pharmaceutical carrier to a patient in need thereof. The pharmaceutical carrier may be solid or liquid.

Applicable solid carriers can include one or more substances which may also act as flavoring agents, lubricants, solubilizers, suspending agents, fillers, glidants, compression aids, binders or tablet-disintegrating agents or an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers may be used in preparing solutions, suspensions, emulsions, syrups and elixirs. The active ingredient of this invention can be dissolved or suspended in a pharmaceutically acceptable liquid carrier such as water, an organic solvent, a mixture of both or pharmaceutically acceptable oils or fat. The liquid carrier can contain other suitable pharmaceutical additives such a solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (particularly containing additives as above, e.g., cellulose derivatives, preferable sodium carboxymethyl cellulose solution), alcohols (including monohydric alcohols and polyhydric alcohols, e.g., glycols) and their derivatives, and oils (e.g., fractionated coconut oil and arachis oil). For parenteral administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. Oral administration may be either liquid or solid composition form.

The compounds of this invention may be administered rectally in the form of a conventional suppository. For administration by intranasal or intrabronchial inhalation or insufflation, the compounds of this invention may be formulated into an aqueous or partially aqueous solution, which can then be utilized in the form of an aerosol. The compounds of this invention may also be administered transdermally through the use of a transdermal patch containing the active compound and a carrier that is inert to the active compound, is non-toxic to the skin, and allows delivery of the agent

for systemic absorption into the blood stream via the skin. The carrier may take any number of forms such as creams and ointments, pastes, gels, and occlusive devices. The creams and ointments may be viscous liquid or semi-solid emulsions of either the oil in water or water in oil type. Pastes comprised of absorptive powders dispersed in petroleum or hydrophilic petroleum containing the active ingredient may also be suitable. A variety of occlusive devices may be used to release the active ingredient into the blood stream such as a semipermeable membrane covering a reservoir containing the active ingredient with or without a carrier, or a matrix containing the active ingredient. Other occlusive devices are known in the literature.

The dosage to be used in the treatment of a specific patient suffering a MMP or TACE dependent condition must be subjectively determined by the attending physician. The variables involved include the severity of the dysfunction, and the size, age, and response pattern of the patient. Treatment will generally be initiated with small dosages less than the optimum dose of the compound. Thereafter the dosage is increased until the optimum effect under the circumstances is reached. Precise dosages for oral, parenteral, nasal, or intrabronchial administration will be determined by the administering physician based on experience with the individual subject treated and standard medical principles.

Preferably the pharmaceutical composition is in unit dosage form, e.g., as tablets or capsules. In such form, the composition is sub-divided in unit dose containing appropriate quantities of the active ingredient; the unit dosage form can be packaged compositions, for example packed powders, vials, ampoules, prefilled syringes or sachets containing liquids. The unit dosage form can be, for example, a capsule or tablet itself, or it can be the appropriate number of any such compositions in package form.

## Claims

1. A compound having the formula **B:**

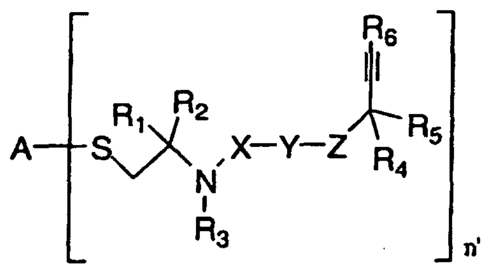

**B**

wherein n' = 1 or 2;
and when n' = 2, then A is absent ;
and when n' = 1, then A is H or $R_{14}C(=W)$- ;

in which formula:

| | |
|---|---|
| W | is oxygen or sulfur; |
| X | is $SO_2$ or -P(O)-$R_{10}$; |
| Y | is aryl or heteroaryl as defined below, with the proviso that X and Z may not be bonded to adjacent atoms of Y; |
| Z | is O, NH, $CH_2$ or S; |
| $R_1$ | is hydrogen, aryl, alkyl of 1-6 carbon atoms, alkenyl of 2-6 carbon atoms, alkynyl of 2-6 carbon atoms; |
| $R_2$ | is hydrogen, aryl or heteroaryl as defined below, cycloalkyl of 3-6 carbon atoms, -$C_5$-$C_8$-cycloheteroalkyl, alkyl of 1-6 carbon atoms, alkenyl of 2-6 carbon atoms, alkynyl of 2-6 carbon atoms, or $CONR_8R_9$; |
| or $R_1$ and $R_2$, | together with the atom to which they are attached, may form a ring wherein $R_1$ and $R_2$ represent a divalent moiety of the formula: |

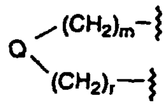 ;

wherein

Q = a carbon-carbon single or double bond, O, S, SO, -N-$R_{11}$, or -$CONR_{15}$;

m = 1-3;

r = 1 or 2, with the proviso that when Q is a bond, r is equal to 2;

Aryl is phenyl or naphthyl optionally substituted by one to two substituents selected from $R_7$, where $R_7$ is as defined below;

Heteroaryl is defined as

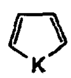 , 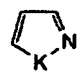 ,, 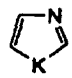 , 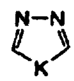 , 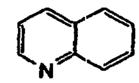 ,

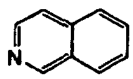 , 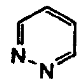 ; 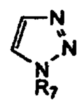 , 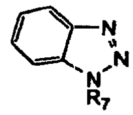 ,

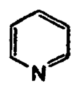 , 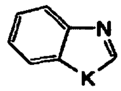 , 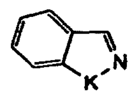 , 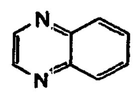 ,

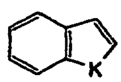 , 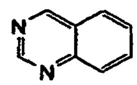 , 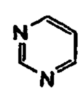 , or 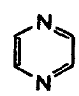 ;

optionally mono- or di- substituted by $R_7$, wherein K is defined as O, S or -$NR_{15}$;

$R_3$ is hydrogen or alkyl of 1-6 carbon atoms;

or $R_1$ and $R_3$, together with the atoms to which they are attached, may form a 5 to 8 membered ring wherein $R_1$ and $R_3$ represent divalent moieties of the formulae:

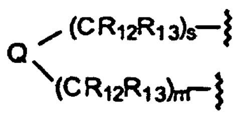 ; and 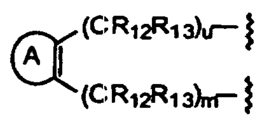

wherein Q and m are as defined above;

A is aryl or heteroaryl;

s is 0-3;

u is 1-4;

$R_4$ and $R_5$ are each, independently, hydrogen, alkyl of 1-6 carbon atoms, -CN, - CCH;

$R_6$ is hydrogen, aryl, heteroaryl, alkyl of 1-6 carbon atoms, alkenyl of 2-6 carbon atoms, alkynyl of 2-6 carbon atoms, cycloalkyl of 3-6 carbon atoms or-$C_5$-$C_8$-cycloheteroalkyl as defined below;

$R_7$ is hydrogen, halogen, alkyl of 1-6 carbon atoms; alkenyl of 2-6 carbon atoms; alkynyl of 2-6 carbon atoms, cycloalkyl of 3-6 carbon atoms, - $OR_8$, -CN, -$COR_8$, perfluoroalkyl of 1-4 carbon atoms, -O-perfluoroalkyl of 1-4 carbon atoms, -$CONR_8R_9$, -$S(O)_nR_8$, - $OPO(OR_8)OR_9$, -$PO(OR_8)R_9$, -OC

(O)$NR_8R_9$, -C(O)$NR_8OR_9$, - $COOR_8$, -$SO_3H$, -$NR_8R_9$, -N[$(CH_2)_2$]$_2NR_8$, -$NR_8COR_9$, -$NR_8COOR_9$, - $SO_2NR_8R_9$, -$NO_2$, -N($R_8$)$SO_2R_9$, -$NR_8CONR_8R_9$, - $NR_8C(=NR_9)NR_8R_9$, -tetrazol-5-yl, -$SO_2NHCN$, -$SO_2NHCONR_8R_9$, phenyl, heteroaryl as defined above, or -$C_5$-$C_8$-cycloheteroalkyl as defined below;

wherein -$NR_8R_9$ may form a pyrrolidine, piperidine, morpholine, thiomorpholine, oxazolidine, thiazolidine, pyrazolidine, piperazine, or azetidine ring;

wherein -$C_5$-$C_8$-cycloheteroalkyl is defined as

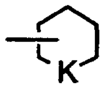 , 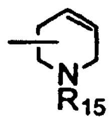 , 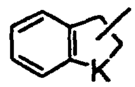 ,

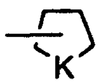 , 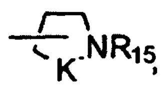, 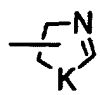 , 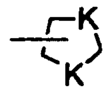 ,

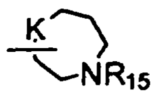 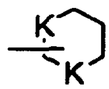 , 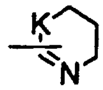 , or 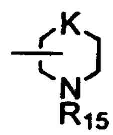 ;

wherein K is defined as above;

| | |
|---|---|
| $R_8$ and $R_9$ | are each, independently, hydrogen, alkyl of 1-6 carbon atoms, cycloalkyl of 3-6 carbon atoms, aryl, heteroaryl or -$C_5$-$C_8$-cycloheteroalkyl; |
| $R_{10}$ | is alkyl of 1-6 carbon atoms, cycloalkyl of 3-6 carbon atoms, aryl or heteroaryl as defined above; |
| $R_{11}$ | is hydrogen, alkyl of 1-6 carbon atoms, cycloalkyl of 3-6 carbon atoms, aryl, heteroaryl, -$S(O)_nR_8$, -$COOR_8$, -$CONR_8R_9$, -$SO_2NR_8R_9$ or -$COR_8$; |
| $R_{12}$ and $R_{13}$ | are independently selected from H, -$OR_8$, -$NR_8R_9$, alkyl of 1-6 carbon atoms, alkenyl of 2-6 carbon atoms, alkynyll of 2-6 carbon atoms, cycloalkyl of 3-6 carbon atoms, aryl, heteroaryl, -$COOR_8$; -$CONR_8R_9$; or $R_{12}$ and $R_{13}$ together form a -$C_3$-$C_6$-cycloalkyl of 3-6 carbon atoms or a -$C_5$-$C_8$-cycloheteroalkyl ring; or $R_{12}$ and $R_{13}$, together with the carbon to which they are attached, form a carbonyl group; with the proviso that $R_{10}$ and $R_{12}$ or $R_{11}$ and $R_{12}$ may form a cycloheteroalkyl ring, wherein cycloheteroalkyl is as defined above, when they are attached to adjacent atoms; |
| $R_{14}$ | is -$OR_8$, -$NR_8R_9$, alkyl of 1-6 carbon atoms, cycloalkyl of 3-6 carbon atoms, aryl or heteroaryl; |
| $R_{15}$ | is hydrogen, aryl, heteroaryl, alkyl of 1-6 carbon atoms or cycloalkyl of 3-6 carbon atoms; |
| and n | is 0-2; |

wherein said alkyl, alkenyl, alkynyl, cycloalkyl may be unsubstituted or mono or poly-substituted with substituents independently selected from $R_7$; or a pharmaceutically acceptable salt thereof.

**2.** A compound according to Claim 1 of formula **B** wherein n'=1, A is H, and Y is phenyl or a pharmaceutically acceptable salt thereof.

**3.** A compound according to Claim 1 or Claim 2 wherein X is $SO_2$; or a pharmaceutically acceptable salt thereof.

**4.** A compound according to any one of Claims I to 3 wherein Z is oxygen; or a pharmaceutically acceptable salt thereof.

**5.** A compound according to any one of Claims 1 to 4 wherein $R_4$ and $R_5$ are hydrogen; or a pharmaceutically acceptable salt thereof.

**6.** A compound according to any one of Claims 1 to 5 wherein $R_6$ is $-CH_2OH$ or methyl; or a pharmaceutically acceptable salt thereof.

**7.** A compound according to Claim 1 wherein $R_1$ is hydrogen, such that this compound has the absolute stereochemistry as shown in structure **1a** below.

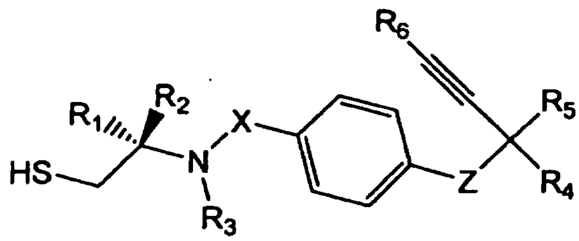

**1a**

**8.** A compound of formula **II,** with the proviso that $R_6$ is not hydrogen and $R_4$, $R_5$ and $R_6$ are as defined in Claim 1.

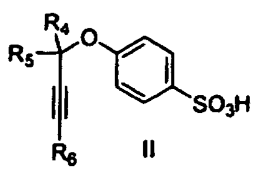

II

**9.** A compound of formula **III,** with the proviso that $R_6$ is not hydrogen wherein J is fluorine, bromine, chlorine, 1,2,4-triazolyl, benzotriazolyl or imidazolyl, and $R_4$, $R_5$ and $R_6$ are as defined in Claim 1.

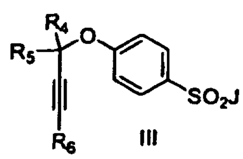

III

**10.** A compound according to Claim 1 which is one of the following:

4-But-2-ynyloxy-N-((1R)-2-mercapto-1-methyl-ethyl)-N-methylbenzenesulfonamide;
(2R)-2- {{[4-(2-Butynyloxy)phenyl]sulfonyl}[2-(4-morpholinyl)ethyl]amino}-3-sulfanylpropanamide, or
4-(2-Butynyloxy)-N-[(1R)-1-methyl-2-sulfanylethyl]-N-[2-(4-morpholinyl) ethyl]benzenesulfonamide.

**11.** A process for preparing compounds of formula B as defined in Claim 1 which comprises one of the following:

a) reacting a compound of formula V:

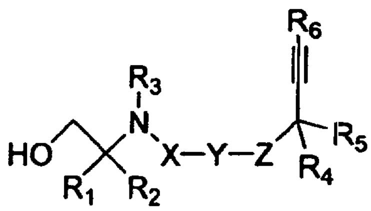

(V)

wherein $R_1$-$R_6$, X, Y and Z are defined in claim 1, with a compound of formula $R_{14}C(W)SH$ under conditions suitable for the Mitsunobu reaction to give a corresponding compound of formula B wherein n'=1 and A is $R_{14}C(=W)$-; or

b) reacting a compound of formula VI:

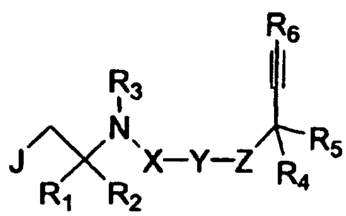

(VI)

wherein $R_1$-$R_6$, X, Y and Z are defined in claim 1, and J is a leaving group, with a compound of formula A'SH where A' is an alkali metal or a $R_{14}C(W)$- group or a salt thereof to give a corresponding compound of formula B wherein n'=1 and A is H or $R_{14}C(=W)$-;
or

c) reacting a compound of formula VIII:

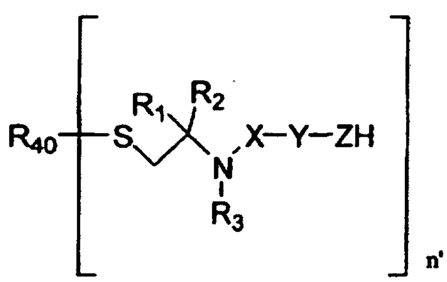

(VII)

wherein $R_1$, $R_2$, $R_3$, X, Y, Z and n' is as defined in claim 1, and when n'=1 $R_{40}$ is H or $R_{14}C(=W)$-, and when n'=2, $R_{40}$ is absent, with a compound of formula VIII:

(VIII)

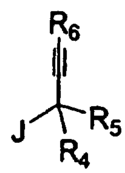

wherein $R_4$, $R_5$, and $R_6$ are as defined above and J is a leaving group such as described above, to give a corresponding compound of formula **B;**
or

d) hydrolysing a compound of formula **B** wherein $R_1$-$R_6$, X, Y and Z are defined in claim 1, n'=1 and A is $R_{14}$C(=W)- , to give a corresponding compound of formula **B** wherein A is H;
or

e) reducing a compound of formula **B** wherein $R_1$-$R_6$, X, Y and Z are defined in claim 1, n'=1 and A is $R_{14}$C(=W)- or n' is 2 , to give a corresponding compound of formula **B** wherein A is H;
or

f) oxidising a compound of formula **B** wherein $R_1$-$R_6$, X, Y and Z are defined in claim 1, n'=1 and A is H , to give a corresponding compound of formula **B** wherein n'=2;
or

g) resolving a mixture (e.g racemate) of optically active isomers of a compound of formula **B** to isolate one enantiomer or diastereomer substantially free of the other enantiomer or diastereomers;
or

h) acidifying a basic compound of formula **B** with a pharmaceutically acceptable acid to give a pharmaceutically acceptable salt.

**12.** Use of a compound according to Claim I in the preparation of a medicament for inhibiting pathological changes mediated by TNF-α converting enzyme (TACE) in a mammal.

**13.** Use according to Claim 12 wherein the condition treated is rheumatoid arthritis, graft rejection, cachexia, inflammation, fever, insulin resistance, septic shock, congestive heart failure, inflammatory disease of the central nervous system, inflammatory bowel disease or HIV infection.

**14.** A pharmaceutical composition comprising a compound as claimed in claim 1 or a salt thereof and a pharmaceutically acceptable carrier.

## Patentansprüche

**1.** Verbindung mit der Formel B:

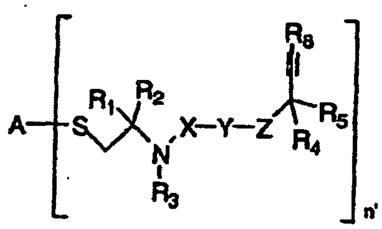

**B**

worin n' = 1 oder 2;
und wenn n' = 2, dann ist A abwesend;
und wenn n' = 1, dann steht A für H oder $R_{14}C(=W)$-;
in welcher Formel:

W für Sauerstoff oder Schwefel steht;
X für $SO_2$ oder -P(O)-$R_{10}$ steht;
Y für Aryl oder Heteroaryl wie unten definiert steht, mit der Maßgabe, dass X und Z nicht an angrenzende Atome von Y gebunden sein sollen;
Z für O, NH, $CH_2$ oder S steht;
$R_1$ für Wasserstoff, Aryl, Alkyl mit 1-6 Kohlenstoffatomen, Alkenyl mit 2-6 Kohlenstoffatomen, Alkinyl mit 2-6 Kohlenstoffatomen steht;
$R_2$ für Wasserstoff, Aryl oder Heteroaryl wie unten definiert, Cycloalkyl mit 3-6 Kohlenstoffatomen, -$C_5$-$C_8$-Cycloheteroalkyl, Alkyl mit 1-6 Kohlenstoffatomen, Alkenyl mit 2-6 Kohlenstoffatomen, Alkinyl mit 2-6 Kohlenstoffatomen oder $CONR_8R_9$ steht; oder
$R_1$ und $R_2$ zusammen mit dem Atom, an welches sie gebunden sind, einen Ring bilden können, worin $R_1$ und $R_2$ eine zweiwertige Komponente der Formel:

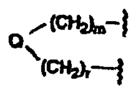 ;

darstellen;
worin

Q = eine Kohlenstoff-Kohlenstoff Einfach- oder Doppelbindung, 0, S, SO, -N-$R_{11}$ oder -$CONR_{15}$;
m = 1-3;
r = 1 oder 2, mit der Maßgabe, dass wenn Q für eine Bindung steht, ist r gleich 2;

Aryl für Phenyl oder Naphthyl steht, gegebenenfalls substituiert durch einen oder zwei Substituenten, ausgewählt aus $R_7$, wobei $R_7$ wie unten definiert ist;
Heteroaryl definiert ist als:

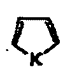 . 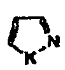 . 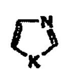 . 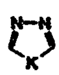 . 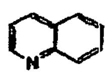 .

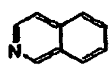 . 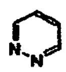 , 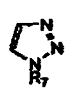 . 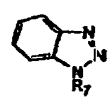 .

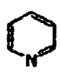 . 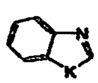 . 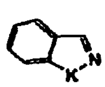 . 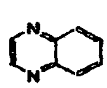 .

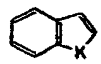 , 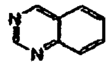 , 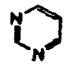 oder 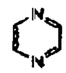 ;

gegebenenfalls mono- oder disubstituiert durch $R_7$, worin K als O, S oder $-NR_{15}$ definiert ist;
$R_3$ für Wasserstoff oder Alkyl mit 1-6 Kohlenstoffatomen steht; oder
$R_1$ und $R_3$ zusammen mit den Atomen, an welche sie gebunden sind, einen 5- bis 8-gliedrigen Ring bilden können, worin $R_1$ und $R_3$ zweiwertige Komponenten der Formeln:

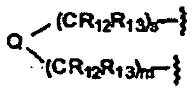 : und 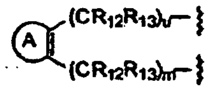

darstellen, worin

Q und m wie oben definiert sind;
A für Aryl oder Heteroaryl steht;
s 0-3 ist;
u 1-4 ist;

$R_4$ und $R_5$ jeweils unabhängig Wasserstoff, Alkyl mit 1-6 Kohlenstoffatomen, -CN, -CCH darstellen;
$R_6$ für Wasserstoff, Aryl, Heteroaryl, Alkyl mit 1-6 Kohlenstoffatomen, Alkenyl mit 2-6 Kohlenstoffatomen, Alkinyl mit 2-6 Kohlenstoffatomen, Cycloalkyl mit 3-6 Kohlenstoffatomen oder $-C_5$-$C_8$-Cycloheteroalkyl wie unten definiert steht;
$R_7$ für Wasserstoff, Halogen, Alkyl mit 1-6 Kohlenstoffatomen; Alkenyl mit 2-6 Kohlenstoffatomen; Alkinyl mit 2-6 Kohlenstoffatomen, Cycloalkyl mit 3-6 Kohlenstoffatomen, $-OR_8$, -CN, $-COR_8$, Perfluoralkyl mit 1-4 Kohlenstoffatomen, -O-Perfluoralkyl mit 1-4 Kohlenstoffatomen, $-CONR_8R_9$, $-S(O)_nR_8$, $-OPO(OR_8)OR_9$, $-PO(OR_8)R_9$, $-OC(O)NR_8R_9$, $-C(O)NR_8OR_9$, $-COOR_8$, $-SO_3H$, $-NR_8R_9$, $-N[(CH_2)_2]_2NR_8$, $-NR_8COR_9$, $-NR_8COOR_9$, $-SO_2NR_8R_9$, $-NO_2$, $-N(R_8)SO_2R_9$, $-NR_8CONR_8R_9$, - $NR_8C(=NR_9)NR_8R_9$, -tetrazol-5-yl, $-SO_2NHCN$, $-SO_2NHCONR_8R_9$, Phenyl, Heteroaryl wie oben definiert oder $-C_5$-$C_8$-Cycloheteroalkyl wie unten definiert steht;

worin $-NR_8R_9$ einen Pyrrolidin-, Piperidin-, Morpholin-, Thiomorpholin-, Oxazolidin-, Thiazolidin-, Pyrazolidin-, Piperazin- oder Azetidinring bilden kann,
worin $-C_5$-$C_8$-Cycloheteroalkyl definiert ist als:

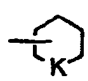 , 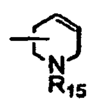 , 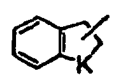 ,

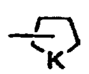 , 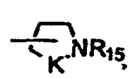 , 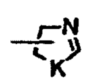 , 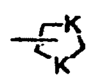 ,

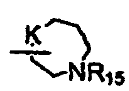 , 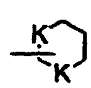 , 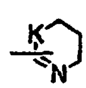 oder 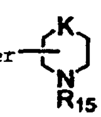 ;

worin K wie oben definiert ist;
$R_8$ und $R_9$ jeweils unabhängig Wasserstoff, Alkyl mit 1-6 Kohlenstoffatomen, Cycloalkyl mit 3-6 Kohlenstoffatomen, Aryl, Heteroaryl oder $-C_5$-$C_8$-Cycloheteroalkyl darstellen;
$R_{10}$ für Alkyl mit 1-6 Kohlenstoffatomen, Cycloalkyl mit 3-6 Kohlenstoffatomen, Aryl oder Heteroaryl wie oben definiert steht;

$R_{11}$ für Wasserstoff, Alkyl mit 1-6 Kohlenstoffatomen, Cycloalkyl mit 3-6 Kohlenstoffatomen, Aryl, Heteroaryl, $-S(O)_nR_8$, $-COOR_8$, $-CONR_8R_9$, $-SO_2NR_8R_9$ oder $-COR_8$ steht;
$R_{12}$ und $R_{13}$ unabhängig ausgewählt werden aus H, $-OR_8$, $-NR_8R_9$, Alkyl mit 1-6 Kohlenstoffatomen, Alkenyl mit 2-6 Kohlenstoffatomen, Alkinyl mit 2-6 Kohlenstoffatomen, Cycloalkyl mit 3-6 Kohlenstoffatomen, Aryl, Heteroaryl, $-COOR_8$, $-CONR_8R_9$; oder $R_{12}$ und $R_{13}$ zusammen ein $-C_3$-$C_6$-Cycloalkyl mit 3-6 Kohlenstoffatomen oder einen $-C_5$-$C_8$-Cycloheteroalkylring bilden; oder $R_{12}$ und $R_{13}$ zusammen mit dem Kohlenstoff, an welchen sie gebunden sind, eine Carbonylgruppe bilden;
mit der Maßgabe, dass $R_{10}$ und $R_{12}$ oder $R_{11}$ und $R_{12}$ einen Cycloheteroalkylring bilden können, wobei Cycloheteroalkyl wie oben definiert ist, wenn sie an angrenzende Atome gebunden sind;
$R_{14}$ für $-OR_8$, $-NR_8R_9$, Alkyl mit 1-6 Kohlenstoffatomen, Cycloalkyl mit 3-6 Kohlenstoffatomen, Aryl oder Heteroaryl steht;
$R_{15}$ für Wasserstoff, Aryl, Heteroaryl, Alkyl mit 1-6 Kohlenstoffatomen oder Cycloalkyl mit 3-6 Kohlenstoffatomen steht; und n für 0-2 steht;

wobei besagtes Alkyl, Alkenyl, Alkinyl, Cycloalkyl nicht substituiert oder mono- oder polysubstituiert sein kann mit Substituenten, unabhängig ausgewählt aus $R_7$;
oder ein pharmazeutisch annehmbares Salz davon.

**2.** Verbindung gemäß Anspruch 1 der Formel B, worin n' = 1, A für H steht und Y für Phenyl steht, oder ein pharmazeutisch annehmbares Salz davon.

**3.** Verbindung gemäß Anspruch 1 oder Anspruch 2, worin X für $SO_2$ steht; oder ein pharmazeutisch annehmbares Salz davon.

**4.** Verbindung gemäß einem der Ansprüche 1 bis 3, worin Z für Sauerstoff steht; oder ein pharmazeutisch annehmbares Salz davon.

**5.** Verbindung gemäß einem der Ansprüche 1 bis 4, worin $R_4$ und $R_5$ Wasserstoff darstellen; oder ein pharmazeutisch annehmbares Salz davon.

**6.** Verbindung gemäß einem der Ansprüche 1 bis 5, worin $R_6$ für $-CH_2OH$ oder Methyl steht; oder ein pharmazeutisch annehmbares Salz davon.

**7.** Verbindung gemäß Anspruch 1, worin $R_4$ für Wasserstoff steht, so dass diese Verbindung die absolute Stereochemie hat, wie in Struktur **1a** unten gezeigt:

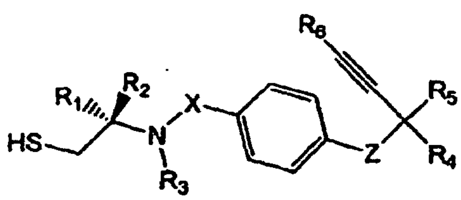

**1a**

**8.** Verbindung der Formel **II,** mit der Maßgabe, dass $R_6$ nicht für Wasserstoff steht und $R_4$, $R_5$ und $R_6$ wie in Anspruch 1 definiert sind:

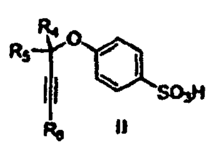

**II**

**9.** Verbindung der Formel **III,** mit der Maßgabe, dass $R_6$ nicht für Wasserstoff steht, worin J für Fluor, Brom, Chlor,

1,2,4-Triazolyl, Benzotriazolyl oder Imidazolyl steht und $R_4$, $R_5$ und $R_6$ wie in Anspruch 1 definiert sind:

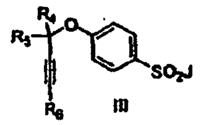

III

**10.** Verbindung gemäß Anspruch 1, welche eine der folgenden ist:

4-But-2-inyloxy-N-((1R)-2-mercapto-1-methyl-ethyl)-N-methylbenzol-sulfonamid;
(2R)-2-{{[4-(2-Butinyloxy)phenyl]sulfonyl}[2-(4-morpholinyl)-ethyl]amino}-3-sulfanylpropanamid oder
4-(2-Butinyloxy)-N-[(1R)-1-methyl-2-sulfanylethyl]-N-[2-(4-morpholinyl)-ethyl]-benzolsulfonamid.

**11.** Verfahren zum Herstellen von Verbindungen der Formel B wie in Anspruch 1 definiert, welches eines der Folgenden umfasst:

a) Umsetzen einer Verbindung der Formel V:

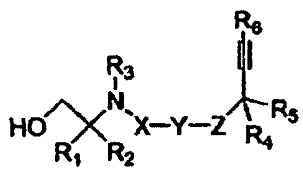

(V)

worin $R_1$-$R_6$, X, Y und Z wie in Anspruch 1 definiert sind, mit einer Verbindung der Formel $R_{14}C(W)SH$ unter Bedingungen, welche für die Mitsunobu-Umsetzung geeignet sind, um eine entsprechende Verbindung der Formel **B** zu ergeben, worin n' = 1 und A für $R_{14}C$ (=W)- steht;
b) Umsetzen einer Verbindung der Formel **VI:**

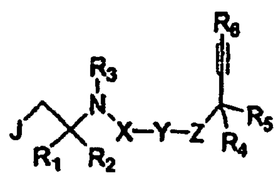

(VI)

worin $R_1$-$R_6$, X, Y und Z wie in Anspruch 1 definiert sind und J für eine Abgangsgruppe steht, mit einer Verbindung der Formel A'SH, wo A' für ein Alkalimetall oder eine $R_{14}C(W)$-Gruppe steht oder ein Salz davon, um eine entsprechende Verbindung der Formel **B** zu ergeben, worin n' = 1 und A für H oder $R_{14}C(=W)$- steht; oder
c) Umsetzen einer Verbindung der Formel **VII:**

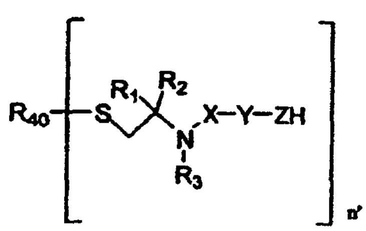

(VII)

worin $R_1$, $R_2$, $R_3$, X, Y, Z und n' wie in Anspruch 1 definiert sind und wenn n' = 1, $R_{40}$ für H oder $R_{14}C(=W)$- steht und wenn n' = 2, $R_{40}$ abwesend ist, mit einer Verbindung der Formel **VIII:**

(VIII)

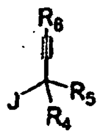

worin $R_4$, $R_5$ und $R_6$ wie oben definiert sind und J für eine Abgangsgruppe steht, wie oben beschrieben, um eine entsprechende Verbindung der Formel **B** zu ergeben; oder

d) Hydrolysieren einer Verbindung der Formel **B**, worin $R_1$-$R_6$, X, Y und Z wie in Anspruch 1 definiert sind, n' = 1 und A für $R_{14}C(=W)$- steht, um eine entsprechende Verbindung der Formel **B** zu ergeben, worin A für H steht; oder

e) Reduzieren einer Verbindung der Formel **B,** worin $R_1$-$R_6$, X, Y und Z wie in Anspruch 1 definiert sind, n' = 1 und A für $R_{14}C$ (=W)- steht oder n' für 2 steht, um eine entsprechende Verbindung der Formel **B** zu ergeben, worin A für H steht; oder

f) Oxidieren einer Verbindung der Formel **B,** worin $R_1$-$R_6$, X, Y und Z wie in Anspruch 1 definiert sind, n' = 1 und A für H steht, um eine entsprechende Verbindung der Formel **B** zu ergeben, worin n' = 2; oder

g) Trennen eines Gemisches (z.B. Racemat) aus optisch aktiven Isomeren einer Verbindung der Formel **B**, um ein Enantiomer oder Diastereomer im Wesentlichen frei vom anderen Enantiomer oder Diastereomer zu isolieren; oder

h) Säuern einer basischen Verbindung der Formel **B** mit einer pharmazeutisch annehmbaren Säure, um ein pharmazeutisch annehmbares Salz zu ergeben.

**12.** Verwenden einer Verbindung gemäß Anspruch 1 bei der Herstellung eines Medikaments zum Hemmen von pathologischen Veränderungen, welche durch TNF-$\alpha$ umwandelndes Enzym (TACE) vermittelt werden, in einem Säuger.

**13.** Verwendung gemäß Anspruch 12, wobei der behandelte Zustand Rheumatoidarthritis, Transplantatabstoßung, Kachexie, Entzündung, Fieber, Insulinresistenz, septischer Schock, kongestives Herzversagen, Entzündungserkrankung des zentralen Nervensystems, entzündliche Darmerkrankung oder HIV-Infektion ist.

**14.** Pharmazeutische Zusammensetzung, welche eine Verbindung wie in Anspruch 1 beansprucht oder ein Salz davon und einen pharmazeutisch annehmbaren Träger umfasst.

## Revendications

**1.** Composé ayant la formule B :

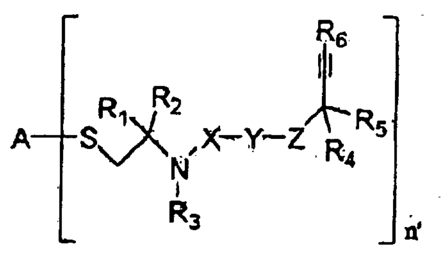

B

dans laquelle n' = 1 ou 2;
et quand n' = 2, alors A est absent;

et quand n' = 1, alors A est H ou $R_{14}C(=W)$-formule dans laquelle :

W est un oxygène ou un soufre;
X est $SO_2$ ou $-P(O)-R_{10}$;
Y est un aryle ou un hétéroaryle tel que défini ci-dessous, avec cette condition que X et Z ne peuvent être liés à des atomes adjacents de Y;
Z est O, NH, $CH_2$ ou S;
$R_1$ est un hydrogène, un aryle, un alkyle de 1 à 6 atomes de carbone, un alcényle de 2 à 6 atomes de carbone, un alcynyle de 2 à 6 atomes de carbone;
$R_2$ est un hydrogène, un aryle ou un hétéroaryle tel que défini ci-dessous, un cycloalkyle de 3 à 6 atomes de carbone, un $-C_5$-$C_8$-cyclohétéroalkyle, un alkyle de 1 à 6 atomes de carbone, un alcényle de 2 à 6 atomes de carbone, un alcynyle de 2 à 6 atomes de carbone, ou $CONR_6R_9$;
ou $R_1$ et $R_2$ ainsi que l'atome auquel ils se rattachent peuvent former un cycle dans lequel $R_1$ et $R_2$ représentent une partie divalente de formule :

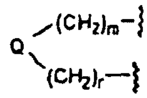 ;

dans laquelle
Q = simple ou double liaison carbone-carbone, O, S, SO, $-N-R_{11}$ ou $-CONR_{15}$;
m = 1-3;
r = 1 ou 2, avec cette condition que lorsque Q est une liaison, r est égal à 2;
L'aryle est un phényle ou un naphtyle éventuellement substitué par un ou deux substituants sélectionnés à partir de $R_7$, où $R_7$ est tel que défini ci-dessous;
L'hétéroaryle est défini comme

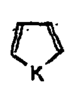 , 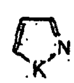 , 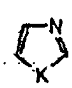 , 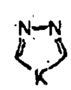 , 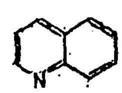 ,

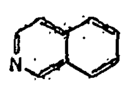 , 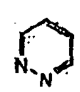 , 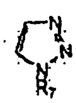 , 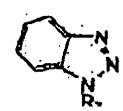 ,

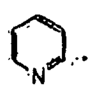 , 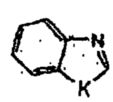 , 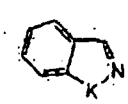 , 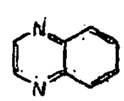 ,

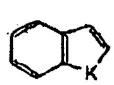 , 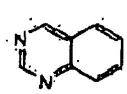 , 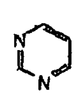 ou 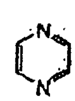 .

éventuellement mono- ou di-substitués par $R_7$, où K est défini comme O, S ou $-NR_{15}$;
$R_3$ est un hydrogène ou un alkyle de 1 à 6 atomes de carbone;
ou $R_1$ et $R_3$ ainsi que les atomes auxquels ils se rattachent peuvent former un cycle de 5 à 8 chaînons, dans lequel $R_1$ et $R_3$ représentent les parties divalentes des formules :

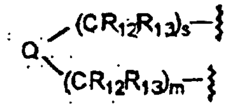 ; et 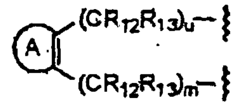

où Q et m sont tels que définis ci-dessus;

A est un aryle ou un hétéroaryle;

s est 0-3;

u est 1-4;

$R_4$ et $R_5$ sont chacun, indépendamment, un hydrogène, un alkyle de 1 à 6 atomes de carbone, -CN, -CCH;

$R_6$ est un hydrogène, un aryle, un hétéroaryle, un alkyle de 1 à 6 atomes de carbone, un alcényle de 2 à 6 atomes de carbone, un alcynyle de 2 à 6 atomes de carbone, un cycloalkyle de 3 à 6 atomes de carbone ou un - $C_5$-$C_6$-cyclohétéroalkyle tel que défini ci-dessous;

$R_7$ est un hydrogène, un halogène, un alkyle de 1 à 6 atomes de carbone, un alcényle de 2 à 6 atomes de carbone, un alcynyle de 2 à 6 atomes de carbone, un cycloalkyle de 3 à 6 atomes de carbone, $-OR_8$, -CN, $COR_8$, un perfluoroalkyle de 1 à 4 atomes de carbone, un -O- perfluoroalkyle de 1 à 4 atomes de carbone, $-CONR_8R_9$, $-S(O)NR_8$, $-OPO(OR_8)OR_9$, $-PO(OR_8)OR_9$, $-OC(O)NR_6R_9$, $-C(O)NR_8OR_9$, $-COOR_8$, $-SO_3H$, $-NR_8R_9$, $-N[(CH_2)_2]_2NR_8$, $-NR_8COR_9$, $-NR_8COOR_9$, $-SO_2NR_8R_9$, $-NO_2$, -N ($R_8$) $SO_2R_9$, - $NR_8CONR_8R_9$, $-NR_8C(=NR_9)NR_8R_9$, un tétrazol-5-yle, $-SO_2NHCN$, - $SO_2NHCONR_8R_9$, un phényle, un hétéroaryle tel que défini ci-dessus, ou un -$C_5$-$C_8$-cyclohétéroalkyle tel que défini ci-dessous;

dans laquelle $-NR_8R_9$ peut former un noyau pyrrolidine, pipéridine, morpholine, thiomorpholine, oxazolidine, thiazolidine, pyrazolidine, pipérazine ou azétidine;

dans laquelle le -$C_5$-$C_8$-cyclohétéroalkyle est défini comme

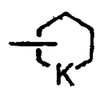 , 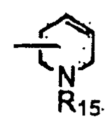 , 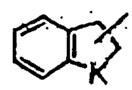 .

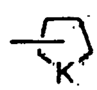 , 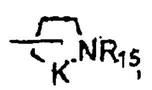 , 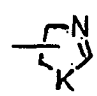 , 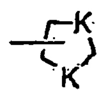 .

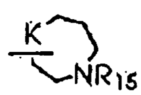 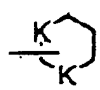 , 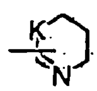 ou 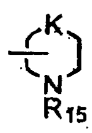 ;

où K est défini comme ci-dessus;

$R_8$ et $R_9$ sont chacun, indépendamment, un hydrogène, un alkyle de 1 à 6 atomes de carbone, un cycloalkyle de 3 à 6 atomes de carbone, un aryle, un hétéroaryle ou un -$C_5$-$C_8$-cyclohétéroalkyle;

$R_{10}$ est un alkyle de 1 à 6 atomes de carbone, un cycloalkyle de 3 à 6 atomes de carbone, un aryle ou un hétéroaryle comme défini ci-dessus;

$R_{11}$ est un hydrogène, un alkyle de 1 à 6 atomes de carbone, un cycloalkyle de 3 à 6 atomes de carbone, un aryle, un hétéroaryle, $-S(O)nR_9$, $-COOR_8$, $-CONR_8R_9$, $-SO_2NR_8R_9$ ou $-COR_8$;

$R_{12}$ et $R_{13}$ sont indépendamment sélectionnés parmi H, $-OR_8$, $-NR_8R_9$, un alkyle de 1 à 6 atomes de carbone, un alcényle de 2 à 6 atomes de carbone, un alcynyle de 2 à 6 atomes de carbone, un cycloalkyle de 3 à 6 atomes de carbone, un aryle, un hétéroaryle, $-COOR_8$, $-CONR_8R_9$; ou $R_{12}$ et $R_{13}$ ensemble forment un - $C_3$-$C_6$-cycloalkyle de 3 à 6 atomes de carbone ou un noyau - $C_5$-$C_8$-cyclohétéroalkyle; ou $R_{12}$ et $R_{13}$ avec le carbone auquel ils se rattachent forment un groupe carbonyle;

avec cette condition que $R_{10}$ et $R_{12}$ ou $R_{11}$ et $R_{12}$ peuvent former un noyau cyclohétéroalkyle, dans lequel le noyau cyclohétéroalkyle est tel que défini ci-dessus, quand ils sont rattachés à des atomes adjacents;

$R_{14}$ est $-OR_6$, $-NR_8R_9$, un alkyle de 1 à 6 atomes de carbone, un cycloalkyle de 3 à 6 atomes de carbone, un

aryle ou un hétéroaryle;
$R_{15}$ est un hydrogène, un aryle, un hétéroaryle, un alkyle de 1 à 6 atomes de carbone ou un cycloalkyle de 3 à 6 atomes de carbone;
et n est 0-2;

dans lequel ledit alkyle, alcényle, alcynyle, cycloalkyle peut être non substitué ou mono- ou poly-substitué par des substituants indépendamment sélectionnés à partir de $R_7$;
ou un sel pharmaceutiquement acceptable de celui-ci.

**2.** Composé selon la revendication 1, de la formule B dans laquelle n'=1, A est H et Y est un phényle, ou un sel pharmaceutiquement acceptable de celui-ci.

**3.** Composé selon la revendication 1 ou la revendication 2, dans lequel X est $SO_2$; ou un sel pharmaceutiquement acceptable de celui-ci.

**4.** Composé selon l'une quelconque des revendications 1 à 3, dans lequel Z est un oxygène; ou un sel pharmaceutiquement acceptable de celui-ci.

**5.** Composé selon l'une quelconque des revendications 1 à 4, dans lequel $R_4$ et $R_5$ sont un hydrogène; ou un sel pharmaceutiquement acceptable de celui-ci.

**6.** Composé selon l'une quelconque des revendications 1 à 5, dans lequel $R_6$ est $-CH_2OH$ ou un méthyle; ou un sel pharmaceutiquement acceptable de celui-ci.

**7.** Composé selon la revendication 1, dans lequel $R_1$ est un hydrogène, tel que ce composé a la stéréochimie absolue présentée dans la structure la ci-dessous.

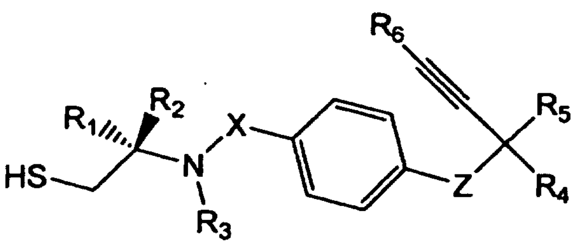

**1a**

**8.** Composé de formule II, avec cette condition que $R_6$ n'est pas un hydrogène et $R_4$, $R_5$ et $R_6$ sont tels que définis dans la revendication 1.

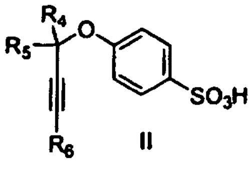

**II**

**9.** Composé de formule III, avec cette condition que $R_6$ n'est pas un hydrogène, dans lequel J est un fluor, un brome, un chlore, un 1,2,4-triazolyle, un benzotriazolyle ou un imidazolyle, et $R_4$, $R_5$ et $R_6$ sont tels que définis dans la revendication 1.

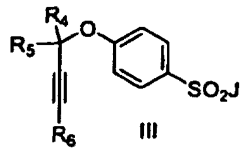

III

10. Composé selon la revendication 1, qui est l'un des suivants :

4-but-2-ynyloxy-N-((1R)-2-mercapto-1-méthyléthyl)-N-méthylbenzènesulfonamide;
(2R)-2-{{[4-(2-butynyloxy)phényl]sulfonyl}[2-(4-morpholinyl)éthyl]amino}-3-sulfanylpropanamide, ou
4-(2-butynyloxy)-N-[(1R)-1-méthyl-2-sulfanyléthyl]-N-[2-(4-morpholinyl)éthyl]benzènesulfonamide.

11. Procédé pour préparer des composés de formule B tel que défini dans la revendication 1, qui comprend l'un des points suivants :

(a) réaction d'un composé de formule V :

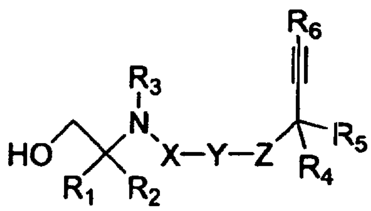

(V)

dans laquelle $R_1$-$R_6$, X, Y et Z sont définis dans la revendication 1, avec un composé de formule $R_{14}C(W)SH$ dans des conditions appropriées pour la réaction de Mitsunobu, pour donner un composé correspondant de formule B dans laquelle n'=1 et A est $R_{14}C(=W)$ -;

(b) réaction d'un composé de formule VI :

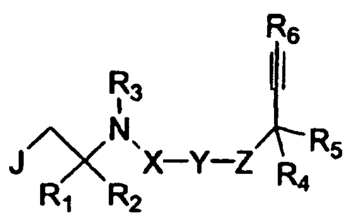

(VI)

dans laquelle $R_1$-$R_6$, X, Y et Z sont définis dans la revendication 1 et J est un groupe partant, avec un composé de formule A'SH où A' est un métal alcalin ou un groupe $R_{14}C(W)$- ou un sel de celui-ci, pour donner un composé correspondant de formule B dans laquelle n'=1 et A est H ou $R_{14}C(=W)$-;

ou

(c) réaction d'un composé de formule VII :

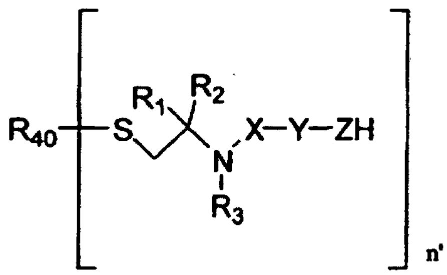

(VII)

dans laquelle $R_1$, $R_2$, $R_3$, X, Y, Z et n' sont tels que définis dans la revendication 1, et quand n'=1 $R_{40}$ est H ou $R_{14}C(W)$-, et quand n'=2, $R_{40}$ est absent, avec un composé de formule VIII :

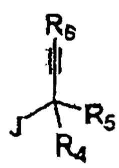

(VIII)

dans laquelle $R_4$, $R_5$ et $R_6$ sont tels que définis ci-dessus et J est un groupe partant tel que décrit ci-dessus, pour donner un composé correspondant de formule B;

ou

(d) hydrolyse d'un composé de formule B dans laquelle $R_1$-$R_6$, X, Y et Z sont définis dans la revendication 1, n'=1 et A est $R_{14}C(=W)$-, pour donner un composé correspondant de formule B dans laquelle A est H;

ou

(e) réduction d'un composé de formule B dans laquelle $R_1$-$R_6$, X, Y et Z sont définis dans la revendication 1, n'=1 et A est $R_{14}C(=W)$- ou n' est 2, pour donner un composé correspondant de formule B dans laquelle A est H;

ou

(f) oxydation d'un composé de formule B dans laquelle $R_1$-$R_6$, X, Y et Z sont définis dans la revendication 1, n'=1 et A est H, pour donner un composé correspondant de formule B dans laquelle n'=2;

ou

(g) résolution d'un mélange (par exemple racémique) d'isomères optiquement actifs d'un composé de formule B pour isoler un énantiomère ou diastéréoisomère en substance dénué de l'autre énantiomère ou diastéréoisomère;

ou

(h) acidification d'un composé basique de formule B avec un acide pharmaceutiquement acceptable pour donner un sel pharmaceutiquement acceptable.

**12.** Utilisation d'un composé selon la revendication 1 pour la préparation d'un médicament pour inhiber des changements pathologiques provoqués par la TACE (enzyme de conversion du TNF-$\alpha$) chez un mammifère.

**13.** Utilisation selon la revendication 12, dans laquelle la pathologie traitée est la polyarthrite rhumatolde, le rejet de greffe, la cachexie, une inflammation, la fièvre, la résistance à l'insuline, un choc septique, l'insuffisance cardiaque congestive, les maladies inflammatoires du système nerveux central, les colopathies inflammatoires ou l'infection au VIH.

**14.** Composition pharmaceutique comprenant un composé selon la revendication 1 ou un sel de celui-ci et un vecteur pharmaceutiquement acceptable.